# EUROPEAN PATENT APPLICATION

(11) **EP 4 667 575 A1**
(43) Date of publication of application: **24.12.2025**
(21) Application number: 24789082.5
(22) Date of filing: 12.04.2024
(51) Int. Cl.: C12N 15/77, C12N 9/10, C12P 13/06, C12P 13/10, C12P 13/12, C12P 13/14, C12P 13/24

(54) **MICROORGANISM WITH ENHANCED PYRUVATE DEHYDROGENASE COMPLEX DIHYDROLIPOYLLYSINE-RESIDUE ACETYLTRANSFERASE ACTIVITY AND METHOD FOR PRODUCING L-AMINO ACIDS USING SAME**

(30) Priority: 12.04.2023 KR 20230048232
(71) Applicant: CJ CheilJedang Corporation, Seoul 04560 (KR)
(72) Inventor: KIM, Ju Eun, Seoul 04560 (KR); LEE, Zeewon, Seoul 04560 (KR); KIM, Ye-Eun, Seoul 04560 (KR); LEE, Hanhyoung, Seoul 04560 (KR); HAN, Seunghee, Seoul 04560 (KR); PARK, Jinsub, Seoul 04560 (KR)
(74) Representative: Meissner Bolte Partnerschaft mbB
(86) International application number: PCT/KR2024/004955
(87) International publication number: WO 2024/215141

(57) **Abstract**

Provided are a microorganism with increased activity of pyruvate dehydrogenase complex dihydrolipoyllysine-residue acetyltransferase, and a method of producing an L-amino acid using the same.

## Description

### [Technical Field]

The present disclosure relates to a microorganism with increased activity of pyruvate dehydrogenase complex dihydrolipoyllysine-residue acetyltransferase, and a method of producing an L-amino acid using the same.

### [Background Art]

Microorganisms of the genus *Corynebacterium* are Gram-positive microorganisms frequently used in the production of L-amino acids.

In order to produce L-amino acids and other useful substances, various studies have been conducted to develop microorganisms with high-efficiency production. For example, for the production of L-amino acids, target material-specific approaches are mainly used, such as a method of increasing the expression of a gene encoding an enzyme mainly involved in L-amino acid biosynthesis in a strain of the genus *Corynebacterium,* or a method of deleting a gene unnecessary the L-amino acid biosynthesis (US 9644009 B2).

### [Disclosure]

### [Technical Problem]

There is a growing need for research on methods capable of efficiently producing an L-amino acid with a high yield.

### [Technical Solution]

One aspect of the present disclosure provides a microorganism with increased activity of pyruvate dehydrogenase complex dihydrolipoyllysine-residue acetyltransferase (AceF).

In one specific embodiment, the microorganism is a microorganism producing an L-amino acid.

In another specific embodiment, the microorganism has increased activity of pyruvate dehydrogenase complex dihydrolipoyllysine-residue acetyltransferase (AceF), as compared to its endogenous activity.

With regard to the microorganism according to any one of the aforementioned specific embodiments, the L-amino acid comprises glutamate-based products, O-acetylhomoserine, homoserine, and methionine.

With regard to the microorganism according to any one of the aforementioned specific embodiments, the L-amino acid is any one or more selected from glutamine, glutamate, ornithine, citrulline, arginine, proline, O-acetylhomoserine, homoserine, and methionine.

With regard to the microorganism according to any one of the aforementioned specific embodiments, the microorganism may be a microorganism of the genus *Corynebacterium.*

With regard to the microorganism according to any one of the aforementioned specific embodiments, the microorganism of the genus *Corynebacterium* may be *Corynebacterium glutamicum.*

With regard to the microorganism according to any one of the aforementioned specific embodiments, the pyruvate dehydrogenase complex dihydrolipoyllysine-residue acetyltransferase may be derived from *Corynebacterium glutamicum.*

With regard to the microorganism according to any one of the aforementioned specific embodiments, the pyruvate dehydrogenase complex dihydrolipoyllysine-residue acetyltransferase may comprise an amino acid sequence of SEQ ID NO: 1 or SEQ ID NO: 32.

With regard to the microorganism according to any one of the aforementioned specific embodiments, the pyruvate dehydrogenase complex dihydrolipoyllysine-residue acetyltransferase may be encoded by a polynucleotide of SEQ ID NO: 2 or SEQ ID NO: 33.

With regard to the microorganism according to any one of the aforementioned specific embodiments, the microorganism may have an increased L-amino acid-producing ability, as compared to an unmodified microorganism.

With regard to the microorganism according to any one of the aforementioned specific embodiments, the microorganism may be a microorganism with increased activity of pyruvate dehydrogenase complex subunit E1, as compared to its endogenous activity.

With regard to the microorganism according to any one of the aforementioned specific embodiments, the pyruvate dehydrogenase complex subunit E1 may comprise an amino acid sequence of SEQ ID NO: 10.

With regard to the microorganism according to any one of the aforementioned specific embodiments, the pyruvate dehydrogenase complex subunit E1 may be encoded by a polynucleotide of SEQ ID NO: 11 or SEQ ID NO: 39.

Another aspect of the present disclosure provides a method of producing an L-amino acid, the method comprising the step of culturing, in a medium, the microorganism with increased activity of pyruvate dehydrogenase complex dihydrolipoyllysine-residue acetyltransferase.

In one specific embodiment, the method may further comprise the step of recovering the L-amino acid from the cultured microorganism, a culture of the microorganism, a fermentation product of the microorganism, or the culture medium.

Still another aspect of the present disclosure provides a composition for producing an L-amino acid, the composition comprising the microorganism with increased activity of pyruvate dehydrogenase complex dihydrolipoyllysine-residue acetyltransferase, as compared to its endogenous activity, a culture of the microorganism, a fermentation product of the microorganism, or a combination of two or more thereof.

Still another aspect of the present disclosure provides use of the microorganism with increased activity of pyruvate dehydrogenase complex dihydrolipoyllysine-residue acetyltransferase, as compared to its endogenous activity, in producing an L-amino acid.

Still another aspect of the present disclosure provides a method of preparing an L-amino acid-producing microorganism, the method comprising modifying a microorganism such that the microorganism has increased activity of pyruvate dehydrogenase complex dihydrolipoyllysine-residue acetyltransferase of the present disclosure, as compared to its endogenous activity.

### [Advantageous Effects]

It is possible to produce an L-amino acid with a high yield by using a microorganism of the present disclosure.

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below. Further, a number of papers and patent documents are referenced and cited throughout this specification. The disclosures of the cited papers and patent documents are incorporated herein by reference in their entirety to further clarify the level and scope of the subject matter to which the present disclosure pertains.

### Definition

As used in the specification and appended claims of the present disclosure, the singular forms ("a", "an", and "the") comprise plural referents unless stated otherwise. Further, unless stated otherwise, plural terms shall comprise the singular. As used in the specification and appended claims of the present disclosure, unless stated otherwise, the use of "or" may be used to comprise "and/or".

As used herein, the term "about" may be presented before a particular numerical value. The term "about" used herein comprises not only the exact number recited after the term, but also a range that is near or close to that number. Considering the context in which the number is presented, it may be determined whether any number is close to or near the particular number presented. For example, the term "about" may refer to a range from -10% to +10% of a numerical value. For another example, the term "about" may refer to a range from -5% to +5% of a given numerical value, but is not limited thereto.

As used herein, the descriptions such as the terms "first, second, third..." "i), ii), iii)..." or "(a), (b), (c), (d)..." are used to distinguish individual components, and when the terms are used in reference to steps of a method, use, or assay, these terms are not limited to being performed continually or sequentially, for example, there may be no time interval between these steps, or they may be performed concurrently, or may be performed several seconds, several minutes, several hours, several days, or several months apart.

As used herein, the term "consisting essentially of" may mean that, in the case where the features of the object claimed herein are not substantially affected by the presence of an unspecified component, the unspecified component may be present.

As used herein, the term "consisting of" means that the total ratio of specific component(s) recited after the term is 100%. The components or features that are recited after the term "consisting of" may be essential or mandatory. In some embodiments, in addition to the components or features recited after the term "consisting of", any other components or features, or non-essential components or features may be excluded.

As used herein, the term "comprising" means the presence of features, steps or components recited after the term, and does not exclude the presence of additional one or more features, steps, or components. The components or features recited after the term "comprising" herein may be essential or mandatory. However, in some embodiments, the term may further comprise any other or non-essential components or features.

### Protein, Polypeptide

As used herein, the term "protein" or "polypeptide" refers to a polymer or oligomer of consecutive amino acid residues. In the present disclosure, "polypeptide," "protein," and "peptide" may be used interchangeably with "amino acid sequence."

In some cases, a protein, polypeptide, or peptide exhibiting activity may be referred to as an "enzyme". In the present disclosure, unless indicated otherwise, amino acid sequences are described in an N-terminal to C-terminal direction.

As used herein, the term "mature polypeptide" means a polypeptide in a form without a signal sequence or propeptide sequence. A mature protein/polypeptide/peptide may be a functional form of a protein/polypeptide/peptide. The mature polypeptide may be in a final form after translation or post-translational modification. Examples of the posttranslational modification comprise N- or C-terminal modifications, glycosylation, phosphorylation, leader sequence removal etc., but are not limited thereto.

With respect to amino acid sequences in the present disclosure, it is apparent that a polypeptide or protein "comprising" an amino acid sequence described by a specific sequence number, a polypeptide or protein "consisting of" an amino acid sequence described by a specific sequence number, or a polypeptide or protein "having" an amino acid sequence described by a specific sequence number may also comprise any polypeptide or protein in which some amino acid(s) is(are) deleted, modified, substituted, conservatively substituted or added, as long as it has the identical or corresponding activity to that of the polypeptide or protein consisting of the amino acid sequence of the corresponding sequence number. For example, the polypeptide or protein may also comprise a polypeptide or protein having addition or deletion of a sequence which does not alter the function of the protein inside or before/after (N-terminus or C-terminus) of the polypeptide or protein sequence, a naturally occurring mutation, a silent mutation thereof, or a conservative substitution, as long as it has the identical or corresponding activity.

In one specific embodiment, a polypeptide (protein) conjugated to an N-terminal signal (or leader) sequence that is co-translationally or post-translationally involved in the polypeptide (protein) translocation, or a polypeptide (protein) conjugated to another sequence or linker so as to enable identification, purification, or synthesis of the polypeptide (protein) may also be comprised in the scope of the polypeptide having the amino acid sequence described by the specific sequence number.

As used herein, the term "conservative substitution" refers to substitution of an amino acid with another amino acid having similar structural and/or chemical properties. Such amino acid substitution may generally occur based on similarity of polarity, charge, solubility, hydrophobicity, hydrophilicity, and/or amphipathic nature of a residue. For example, positively charged (basic) amino acids comprise arginine, lysine, and histidine; negatively charged (acidic) amino acids comprise glutamic acid and aspartic acid; amino acids having a nonpolar side chain (nonpolar amino acids) comprise glycine, alanine, valine, leucine, isoleucine, methionine, phenylalanine, tryptophan, and proline; amino acids having a polar or hydrophilic side chain (polar amino acids) comprise serine, threonine, cysteine, tyrosine, asparagine, and glutamine. For another example, amino acids may be classified into amino acids with electrically charged side chains (electrically charged amino acids) such as arginine, lysine, histidine, glutamic acid, and aspartic acid, and amino acids with uncharged side chains (uncharged amino acids; also called neutral amino acids) such as glycine, alanine, valine, leucine, isoleucine, methionine, phenylalanine, tryptophan, proline, serine, threonine, cysteine, tyrosine, asparagine, and glutamine. For still another example, phenylalanine, tryptophan, and tyrosine may be classified as aromatic amino acids. For still another example, valine, leucine, and isoleucine may be classified as branched amino acids. For still another example, 20 types of amino acids are classified according to size, starting from the amino acid group with relatively small volume, the amino acids may be classified into five groups; glycine, alanine, serine; cysteine, proline, threonine, aspartic acid, asparagine; valine, histidine, glutamic acid, glutamine; isoleucine, leucine, methionine, lysine, arginine; and phenylalanine, tryptophan, tyrosine. However, they are not necessarily limited thereto. Usually, conservative substitution may hardly affect or not affect activity of polypeptides.

### Polynucleotide

As used herein, the term "gene" means a polynucleotide that encodes a polypeptide and a polynucleotide including regions upstream and downstream of the coding region. In some embodiments, a gene may have a sequence (intron) inserted between each coding region (exon).

As used herein, the term "polynucleotide, nucleic acid, or nucleic acid molecule" refers to a DNA (e.g., cDNA or genomic DNA) or RNA (e.g., mRNA) strand of a certain length or longer as a polymer of nucleotides in which nucleotide monomers are connected in a long chain form by covalent bonds.

### Homology, Identity

As used herein, the term "homology" or "identity" refers to a degree of similarity between corresponding sequences in two given amino acid or base sequences, and may be expressed as a percentage. The terms homology and identity may often be used interchangeably with each other.

The sequence homology or identity of a conserved polynucleotide or polypeptide is determined by standard alignment algorithms, and the default gap penalty established by a program to be used may be used together. Substantially, homologous or identical sequences may generally hybridize under moderately or highly stringent conditions to the entirety of the sequence or at least about 50%, 60%, 70%, 80%, or 90% of the full-length. It is apparent that hybridization also comprises hybridization of a polynucleotide with a polynucleotide comprising a general codon or a codon in consideration of codon degeneracy.

Whether or not any two polynucleotide or polypeptide sequences have homology, similarity, or identity may be determined using known computer algorithms such as the "FASTA" program, for example, using default parameters as in Pearson et al (1988) [Proc. Natl. Acad. Sci. USA 85]: 2444. Alternatively, it may be determined using the Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (comprising GCG program package ((Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO et al.](1988) SIAM J Applied Math 48: 1073). For example, BLAST of the National Center for Biotechnology Information or ClustalW may be used to determine the homology, similarity, or identity.

The homology, similarity, or identity between polynucleotides or polypeptides may be determined by comparing sequence information using the GAP computer program as introduced by, for example, Needleman et al. (1970), J Mol Biol. 48:443, as disclosed by Smith and Waterman, Adv. Appl. Math (1981) 2:482. Briefly, the GAP program defines the homology, similarity, or identity as the value obtained by dividing the number of similarly aligned symbols (i.e., nucleotides or amino acids) by the total number of the symbols in the shorter of the two sequences. The default parameters for the GAP program may comprise: (1) unitary matrices (comprising a value 1 for identity and a value 0 for non-identity), PAM Matrix (see those described by Schwartz and Dayhoff, eds., Atlas Of Protein Sequence And Structure, National Biomedical Research Foundation (1978)), and the weighted comparison matrix of Gribskov et al(1986) Nucl. Acids Res. 14: 6745 (or EDNAFULL (EMBOSS version of NCBI NUC4.4) substitution matrix); (2) a penalty of 3.0 for each gap and an additional 0.10 penalty for each symbol in each gap (or a gap open penalty of 10 and a gap extension penalty of 0.5); and (3) no penalty for end gaps.

Further, whether or not any two polynucleotide sequences have homology, similarity, or identity may be identified by comparing the sequences in a Southern hybridization experiment under stringent conditions as defined, and appropriate hybridization conditions defined are within the skill of the art, and may be determined by a method well known to those skilled in the art (e.g., J. Sambrook et al., Molecular Cloning, A Laboratory Manual; F.M. Ausubel et al., Current Protocols in Molecular Biology, John Wiley & Sons, Inc., New York), but are not limited thereto.

As used herein, the term "stringent conditions" means conditions that enable specific hybridization between polynucleotides. These conditions are specifically described in documents (see Sambrook et al., supra, 9.50-9.51, 11.7-11.8). Examples thereof comprise conditions in which polynucleotides having higher homology or identity, i.e., polynucleotides having 60% or more, 70% or more, 75% or more, 80% or more, 85% or more, 90% or more, 95% or more, 96% or more, 97% or more, 98% or more, or 99% or more homology or identity, are hybridized with each other while polynucleotides having lower homology or identity are not hybridized with each other, or conditions in which washing is performed once, specifically, twice to three times at a salt concentration and temperature equivalent to 60°C, 1×SSC, 0.1% SDS, specifically 60°C, 0.1×SSC, 0.1% SDS, more specifically, 68°C, 0.1×SSC, 0.1% SDS, which are washing conditions for common Southern hybridization.

The hybridization requires that two nucleotides have complementary sequences, although mismatches between bases are allowed depending on the stringency of hybridization. The term "complementary" is used to describe the relation between nucleotide bases capable of being hybridized with each other. For example, with regard to DNA, adenine is complementary to thymine and cytosine is complementary to guanine. Hence, the polynucleotide of the present disclosure may also comprise substantially similar base sequences as well as isolated nucleic acid fragments that are complementary to the entire sequence.

For example, polynucleotides having homology or identity to the polynucleotide of the present disclosure may be detected using the hybridization conditions comprising a hybridization step at a Tm value of 55°C under the above-described conditions. Further, the Tm value may be 60°C, 63°C, or 65°C, but is not limited thereto, and may be appropriately adjusted by those skilled in the art depending on the purpose thereof.

The appropriate stringency to hybridize the polynucleotide depends on the length and degree of complementarity of the polynucleotide, and the variables are well known in the art (e.g., J. Sambrook et al., supra).

### Nucleic acid construct, Vector, Transformation

As used herein, the term "nucleic acid construct" refers to a single- or double-stranded nucleic acid molecule, which comprises one or more regulatory sequences, and which is artificially synthesized, or engineered to comprise a specific sequence in a manner that does not exist in nature, or isolated from nature.

As used herein, the term "vector" means a DNA construct for delivering a target polynucleotide into a suitable host or host cell, for example, those comprising a nucleotide sequence of a polynucleotide encoding a target polypeptide which is operably linked to a suitable expression regulatory region (or expression control sequence) so that the target polypeptide may be expressed in a suitable host. The expression regulatory region may comprise a promoter capable of initiating transcription, any operator sequence for controlling the transcription, a sequence encoding a suitable mRNA ribosome binding site, and a sequence controlling termination of transcription and translation. The vector may be transformed into a suitable host cell, and then replicate or function independently of the host genome, or may be integrated into the genome itself.

The vector used in the present disclosure is not particularly limited, but any vector known in the art may be used. Examples of commonly used vectors may comprise natural or recombinant plasmids, cosmids, viruses, and bacteriophages. For example, pWE15, M13, MBL3, MBL4, IXII, ASHII, APII, t10, t11, Charon4A, Charon21A, etc. may be used as a phage vector or a cosmid vector, and pDZ system, pDC system, pBR system, pUC system, pBluescript II system, pGEM system, pTZ system, pCL system, pET system, etc. may be used as a plasmid vector. For example, a pDZ, pDC, pACYC177, pACYC184, pCL, pECCG117, pUC19, pBR322, pMW118, pCC1BAC, pDCM2 (WO2021-187781 A1) vector, etc. may be used.

For example, a target polynucleotide may be inserted into a chromosome through a vector for intracellular chromosome insertion. Insertion of the polynucleotide into the chromosome may be performed by any method known in the art, for example, homologous recombination, but is not limited thereto. The vector may further comprise a selection marker for the confirmation of chromosome insertion. The selection marker is for selecting the cells transformed with vectors, i.e., for confirming the insertion of a target nucleic acid molecule, and markers that confer selectable phenotypes such as drug resistance, auxotrophy, resistance to cytotoxic agents, or expression of surface polypeptides may be used. In an environment treated with a selective agent, only cells expressing the selection marker survive or exhibit other phenotypic traits, and thus transformed cells may be selected.

As used herein, the term "transformation" means that a vector comprising a target polynucleotide is introduced into a host cell so that the genetic traits of the host cell are changed. The transformed polynucleotide may be located either by being inserted into the chromosome of the host cell or located outside the chromosome. Further, the polynucleotide comprises DNA or RNA. The polynucleotide may be introduced in an appropriate form depending on the purpose of introduction. For example, the polynucleotide for expressing the target polypeptide may be introduced into a host cell in the form of an expression cassette, which is a gene construct comprising all elements required for self-expression. The expression cassette may usually comprise a promoter operably linked to the polynucleotide, a transcription termination signal, a ribosome binding site, and a translation termination signal. The expression cassette may be in the form of an expression vector capable of self-replicating. Further, the polynucleotide may be introduced into a host cell in its own form and operably linked to a sequence required for expression in the host cell, but is not limited thereto.

As used herein, the term "operably linked" refers to a configuration of placing a regulatory sequence in an appropriate position to direct expression of a coding sequence. Thus, the term "operably linked" comprises an attachment or linking between a regulatory region of a functional domain having a known or desired activity such as a promoter, a terminator, a signal sequence, or an enhancer, and a target (gene or polypeptide) such that the expression, secretion, or function of the target (gene or polypeptide) may be regulated in accordance with the known or desired activity. For example, the term means that the polynucleotide sequence is functionally linked to a promoter sequence that initiates and mediates transcription of the polynucleotide encoding the polypeptide.

As used herein, the term "expression" comprises any step involved in production of a polypeptide, e.g., transcription, post-transcriptional modification, translation, post-translational modification, and secretion, but is not limited thereto.

As used herein, the term "expression vector" refers to a linear or circular nucleic acid molecule comprising a target polynucleotide sequence and an operably linked regulatory sequence for expression thereof.

As used herein, the term "regulatory sequence" refers to a polynucleotide sequence required for the regulation of expression of a target polynucleotide sequence. Each regulatory sequence may be native (derived from the same origin) or foreign (derived from different genes) to the coding sequence, or a mutant sequence thereof or other artificial sequence. Examples of the regulatory sequence may comprise a leader sequence, a polyadenylation sequence, a propeptide sequence, a promoter, a signal peptide sequence, an operator sequence, a ribosome binding site-encoding sequence, and a sequence terminating transcription and translation. A minimum unit of the regulatory sequence may comprise a promoter and a sequence terminating transcription and translation.

With regard to a cell, polynucleotide, polypeptide, or vector, the term "recombinant" used herein refers to the cell, polynucleotide, polypeptide, or vector modified by introduction of a heterologous nucleic acid or polypeptide or alteration of a native polynucleotide or polypeptide, or a cell derived from the modified cell. Thus, for example, recombinant cells may express genes that are not found within the native (non-recombinant) form of the cells, or may express native genes that are otherwise abnormally expressed, underexpressed or not expressed at all.

### Microorganism

As used herein, the term "microorganism (or strain)" comprises all of wild-type microorganisms, or prokaryotic or eukaryotic microorganisms in which genetic modification naturally or artificially occurs, and it may be a microorganism in which a specific mechanism is weakened or enhanced due to insertion of a foreign gene or an activity enhancement or inactivation of an endogenous gene, and it may be a microorganism comprising genetic modification for the production of a desired polypeptide, protein, or product. As used herein, the "microorganism" and "strain" have the same meaning and may be used interchangeably without limitation.

For example, the microorganism of the present disclosure may be a microorganism (e.g., recombinant strain) with increased activity of pyruvate dehydrogenase complex dihydrolipoyllysine-residue acetyltransferase, as compared to its endogenous activity, but is not limited thereto.

As used herein, the term "microorganism having the L-amino acid-producing ability" refers to a microorganism capable of producing an L-amino acid within a living organism, and may comprise both a microorganism prepared by providing the L-amino acid-producing ability for a microorganism inherently not having the L-amino acid-producing ability, or a microorganism that inherently has the L-amino acid-producing ability. The L-amino acid-producing ability may be conferred or enhanced by species improvement.

As used herein, the term "unmodified microorganism (strain)" does not exclude microorganisms (strains) comprising a mutation that may occur naturally, and may be a wild-type microorganism (strain) or a natural microorganism (strain) itself or may be a microorganism (strain) before the trait is changed by genetic variation due to natural or artificial factors. The "unmodified microorganism (strain)" may be used interchangeably with "microorganism (strain) before being modified", "unvaried microorganism (strain)", "parent microorganism", "parent strain", "wild-type microorganism (strain)", "reference microorganism (strain)" or "standard microorganism (strain)". Further, in the present disclosure, the unmodified microorganism may refer to a microorganism, in which the activity of the pyruvate dehydrogenase complex dihydrolipoyllysine-residue acetyltransferase of the present disclosure is not increased, as compared to its endogenous activity, or before the activity is increased, but is not limited thereto.

Further, in the present disclosure, the unmodified microorganism may be a microorganism comprising an amino acid sequence consisting of SEQ ID NO: 1 or 32; or a polynucleotide consisting of SEQ ID NO: 2 or SEQ ID NO: 33, but is not limited thereto.

### Increase of polypeptide activity

As used herein, the term "increase" of the polypeptide activity means that the activity of a polypeptide in a host cell is enhanced, as compared with its endogenous activity. The increase may be used interchangeably with the terms such as activation, up-regulation, overexpression, enhancement, etc. The host cell may be a prokaryotic or eukaryotic microorganism.

The increase of the polypeptide activity may comprise both cases where a polypeptide activity not originally possessed by a host microorganism is exhibited, or the enhanced polypeptide activity is exhibited, as compared to its endogenous activity or the activity before modification.

For example, "the activity not originally possessed is exhibited" may be caused by "introduction of a polypeptide (protein)", but is not limited thereto. The introduction of the polypeptide (protein) means that a gene not originally possessed by a microorganism is expressed within the microorganism to exhibit the activity of a specific polypeptide (protein) or to exhibit the increased or enhanced activity as compared to endogenous activity of the corresponding polypeptide (protein) or the activity before modification. For example, a polynucleotide encoding a specific polypeptide (protein) may be introduced into a chromosome in a microorganism, or a vector comprising the polynucleotide encoding the specific polypeptide (protein) may be introduced into the microorganism, thereby exhibiting its activity.

The "endogenous activity" means the activity of a specific polypeptide originally possessed by a microorganism before the trait is changed or an unmodified microorganism, when the trait is changed by genetic variation due to natural or artificial factors. This may be used interchangeably with "activity before modification".

The fact that the activity of a polypeptide is enhanced as compared to the endogenous activity means that the activity of the polypeptide is improved as compared to the activity and/or concentration (expression level) of the specific polypeptide originally possessed by a microorganism before the trait is changed or an unmodified microorganism.

For example, the increase indicates that the activity of the corresponding protein/polypeptide originally absent appears, or its activity or concentration is generally increased to about 1%, about 10%, about 25%, about 50%, about 75%, about 100%, about 150%, about 200%, about 300%, about 400% or about 500%, up to about 1000% or about 2000% or more, based on the activity or concentration in the initial microbial strain, but is not limited thereto.

The increase of the activity of the polypeptide may be achieved through the introduction of a foreign polypeptide or the increase of the activity of the endogenous polypeptide. The increase of the activity of the polypeptide may be confirmed by an increase in the degree of activity and the expression level of the corresponding polypeptide, or an increase in the amount of a product due to the activity of the corresponding polypeptide.

For the increase of the activity of the polypeptide, various methods well known in the art may be applied, and the method is not limited as long as the activity of the target polypeptide may be increased as compared to that of the microorganism before modification. Specifically, genetic engineering and/or protein engineering well known to those skilled in the art, which are routine methods of molecular biology, may be used, but the method is not limited thereto (e.g., Sitnicka et al. Functional Analysis of Genes. Advances in Cell Biology. 2010, Vol. 2. 1-16, Sambrook et al. Molecular Cloning 2012, etc.).

Specifically, the increase of the activity of the polypeptide of the present disclosure may be:
1) increase in the intracellular copy number of the polynucleotide encoding the polypeptide;
2) modification of a gene expression regulatory region on a chromosome encoding the polypeptide (e.g., occurrence of variations in the expression regulatory region, replacement with a sequence having stronger activity, or insertion of a sequence having stronger activity);
3) modification of a nucleotide sequence encoding a start codon or 5'-UTR region of the gene transcript encoding the polypeptide;
4) modification of the amino acid sequence of the polypeptide to increase the activity of the polypeptide;
5) modification of the polynucleotide sequence encoding the polypeptide to increase the activity of the polypeptide (e.g., modification of the polynucleotide sequence of the polypeptide gene to encode the polypeptide that has been modified to increase the activity of the polypeptide);
6) introduction of a foreign polypeptide exhibiting the activity of the polypeptide or a foreign polynucleotide encoding the same;
7) codon optimization of the polynucleotide encoding the polypeptide;
8) analysis of the tertiary structure of the polypeptide to select and to modify or chemically modify the exposed site;
9) controlling of intracellular localization of the protein (polypeptide); or
10) a combination of two or more selected from 1) to 9), but is not particularly limited thereto.

For example, 1) the increase in the intracellular copy number of the polynucleotide encoding the polypeptide may be achieved by the introduction, into a host cell, of a vector comprising the polynucleotide encoding the polypeptide, which is operably linked to an appropriate regulatory sequence. The vector may be a vector that is able to replicate and function independently of the host. Alternatively, the increase may be achieved by the introduction of one copy or two or more copies of the polynucleotide encoding the polypeptide, which is operably linked to an appropriate regulatory sequence, into the chromosome in a host cell. The introduction into the chromosome may be performed by the introduction, into a host cell, of a vector capable of inserting the polynucleotide into the chromosome in the host cell, but is not limited thereto. The vector is as described above. The regulatory sequence may be native (derived from the same origin) or foreign (derived from different genes) to the coding polynucleotide sequence, or a mutant sequence thereof or other artificial sequence, and may induce expression of the polynucleotide in the host cell.

2) The replacement of a gene expression regulatory region (expression regulatory sequence) on the chromosome encoding the polypeptide with a sequence with strong activity may be, for example, the occurrence of mutation on the sequence through deletion, insertion, substitution, or a combination thereof, or the replacement with a sequence having stronger activity, to further enhance the activity of the expression regulatory region. The expression regulatory region may comprise, but is not particularly limited to, a promoter, an operator sequence, a sequence encoding a ribosome binding site, a sequence controlling the termination of transcription and translation, etc. For example, it may be the replacement of the original promoter with a stronger promoter, but is not limited thereto.

Examples of the known stronger promoter may comprise cj1 to cj7 promoters (US Patent No. 7662943 B2), lac promoter, trp promoter, trc promoter, tac promoter, lambda phage PR promoter, PL promoter, tet promoter, gapA promoter, SPL7 promoter, SPL13 (sm3) promoter (US Patent No. 10584338 B2), O2 promoter (US Patent No. 10273491 B2), tkt promoter, yccA promoter, etc., but are not limited thereto.

3) The modification of a nucleotide sequence encoding a start codon or 5'-UTR region of the gene encoding the polypeptide may be, for example, replacing with another start codon having a higher polypeptide expression rate than the endogenous start codon, but is not limited thereto.

Further, 4) and 5) the modification of the amino acid sequence or polynucleotide sequence of the polypeptide may be occurrence of mutation on the sequence through deletion, insertion, non-conservative or conservative substitution, or a combination thereof in the amino acid sequence of the polypeptide or the polynucleotide sequence encoding the polypeptide, or the replacement with an amino acid sequence or polynucleotide sequence improved to have stronger activity or an amino acid sequence or polynucleotide sequence improved to have increased activity, in order to increase activity of the polypeptide, but is not limited thereto. Specifically, the replacement may be performed by inserting the polynucleotide into the chromosome by homologous recombination, but is not limited thereto. The vector used herein may further comprise a selection marker for checking the insertion of the chromosome. The selection marker is as described above.

6) The introduction of a foreign polynucleotide exhibiting the activity of the polypeptide may be the introduction, into a host cell, of a foreign polynucleotide encoding a polypeptide exhibiting the identical/similar activity to the polypeptide. The foreign polynucleotide is not limited to the origin or sequence thereof as long as the foreign polynucleotide exhibits the identical/similar activity to the polypeptide. The method used in the introduction may be performed by any known transformation method appropriately selected by a person skilled in the art, and through the expression of the introduced polynucleotide in the host cell, the polypeptide is produced and the activity thereof may be increased.

7) The codon optimization of the polynucleotide encoding the polypeptide may be the codon optimization of an endogenous polynucleotide to increase transcription or translation thereof in a host cell, or the codon optimization of a foreign polynucleotide to allow optimized transcription or translation thereof in a host cell.

8) The modification or chemical modification of an exposed site selected by analysis of a tertiary structure of the polypeptide may be, for example, the modification or chemical modification of an exposed site to be modified or chemically modified by comparing sequence information of a polypeptide to be analyzed with a database that stores sequence information of known proteins, determining a template protein candidate according to similarity of the sequences, and identifying the structure based thereon.

9) The controlling of intracellular localization of the protein (polypeptide) may mean targeting the protein (polypeptide) to a specific organelle within cells or to a specific intracellular space, for example, targeting to the periplasm or cytoplasm through the addition or removal of a leader sequence that functions in targeting the protein (polypeptide), but is not limited thereto.

Such increase of the activity of the polypeptide may mean that the activity or concentration of the corresponding polypeptide is increased relative to the activity or concentration of the polypeptide expressed in the wild-type or the microorganism before modification, or that the amount of a product produced from the corresponding polypeptide is increased, but is not limited thereto.

### Weakening of polypeptide activity

As used herein, the term "weakening" of the activity of a polypeptide (for example, comprising a protein specified by the name of each enzyme) has a concept encompassing all of the reduction of activity or the absence of activity, as compared to the endogenous activity. The weakening may be used interchangeably with the terms such as inactivation, deficiency, down-regulation, decrease, reduction, attenuation, etc.

The weakening may also comprise a case where the activity of the polypeptide itself is reduced or eliminated due to the mutation, etc. of the polynucleotide encoding the polypeptide, as compared to the activity of the polypeptide originally possessed by the microorganism, a case where the overall activity and/or concentration (expression level) of the polypeptides in the cell is low due to the inhibition of the expression of a gene of a polynucleotide encoding the polypeptide or the inhibition of translation into the polypeptide, as compared to the native strain, a case where the expression of the polynucleotide is not made, and/or a case where the polypeptide has no activity even though the polynucleotide is expressed. The "inactivation, deficiency, decrease, down-regulation, reduction, or attenuation" of the activity of a polypeptide compared to the endogenous activity thereof means that the activity of the polypeptide is lowered compared to the activity of a specific polypeptide originally possessed by a parent strain before the trait is changed or an unmodified microorganism.

Such weakening of the activity of the polypeptide may be performed by any method known in the art, but is not limited thereto, and may be achieved by applying various methods well known in the art (e.g., Nakashima N et al., Bacterial cellular engineering by genome editing and gene silencing. Int J Mol Sci. 2014;15(2):2773-2793, Sambrook et al. Molecular Cloning 2012, etc.).

Specifically, the weakening of the activity of the polypeptide of the present disclosure may be achieved by:
1) deletion of the entirety or a part of the gene encoding the polypeptide;
2) modification of an expression regulatory region (or expression regulatory sequence) to reduce the expression of a gene encoding the polypeptide;
3) modification of the amino acid sequence constituting the polypeptide to eliminate or weaken the activity of the polypeptide (e.g., deletion/substitution/addition of one or more amino acids in the amino acid sequence);
4) modification of the gene sequence encoding the polypeptide to eliminate or weaken the activity of the polypeptide (e.g., deletion/substitution/addition of one or more nucleotides in the nucleotide sequence of the polypeptide gene to encode the polypeptide which is modified to eliminate or weaken the activity of the polypeptide);
5) modification of a nucleotide sequence encoding a start codon or 5'-UTR region of the gene transcript encoding the polypeptide;
6) introduction of an antisense oligonucleotide (e.g., antisense RNA) complementarily binding to the gene transcript encoding the polypeptide;
7) addition of a sequence complementary to the Shine-Dalgarno sequence of the gene encoding the polypeptide to the upstream of the Shine-Dalgarno sequence in order to form a secondary structure that makes the attachment of ribosomes impossible;
8) addition of a promoter, which is to be reverse-transcribed, to the 3' end of the open reading frame (ORF) of the gene sequence encoding the polypeptide (reverse transcription engineering, RTE);
9) controlling of intracellular localization of the protein (polypeptide); or
10) combination of two or more selected from 1) to 9), but is not particularly limited thereto.

For example,
1) the deletion of a part or the entirety of the gene encoding the polypeptide may be elimination of the entirety of the polynucleotide encoding an endogenous target protein in the chromosome, replacement with a polynucleotide having deletion of some nucleotides, or replacement with a marker gene.

Further, 2) the modification of an expression regulatory region (or expression regulatory sequence) may be occurrence of mutation on the expression regulatory region (or expression regulatory sequence) through deletion, insertion, non-conservative or conservative substitution, or a combination thereof, or the replacement with a sequence having weaker activity. The expression regulatory region comprises a promoter, an operator sequence, a sequence encoding a ribosomal binding site, and a sequence for controlling the termination of transcription and translation, but is not limited thereto.

3) and 4) The modification of the amino acid sequence or the polynucleotide sequence may be occurrence of mutation on the sequence through deletion, insertion, non-conservative or conservative substitution, or a combination thereof in the amino acid sequence of the polypeptide or the polynucleotide sequence encoding the polypeptide, or a replacement with an amino acid sequence or polynucleotide sequence improved to have weaker activity or an amino acid sequence or polynucleotide sequence improved to have no activity, so as to weaken activity of the polypeptide, but is not limited thereto. For example, the expression of the gene may be inhibited or weakened by introducing mutation into the polynucleotide sequence to form a stop codon, but is not limited thereto.

5) The modification of a nucleotide sequence encoding a start codon or 5'-UTR region of the gene transcript encoding the polypeptide may be, for example, the substitution with a nucleotide sequence encoding, rather than an endogenous start codon, another start codon having a lower expression rate of the polypeptide, but is not limited thereto.

6) The introduction of an antisense oligonucleotide (e.g., antisense RNA) complementarily binding to the gene transcript encoding the polypeptide may be referred to, for example, a literature [Weintraub, H. et al., Antisense-RNA as a molecular tool for genetic analysis, Reviews - Trends in Genetics, Vol. 1(1) 1986].

7) The addition of a sequence complementary to the Shine-Dalgarno sequence of the gene encoding the polypeptide to the upstream of the Shine-Dalgarno sequence so as to form a secondary structure that makes the attachment of ribosomes impossible may be making mRNA translation impossible or reducing the rate thereof.

Further, 8) the addition of a promoter, which is to be reverse-transcribed, to the 3' end of the open reading frame (ORF) of the gene sequence encoding the polypeptide (reverse transcription engineering, RTE) may be making an antisense nucleotide complementary to the gene transcript encoding the polypeptide to weaken the activity thereof.

9) The controlling of intracellular localization of the protein (polypeptide) may mean targeting the protein (polypeptide) to a specific organelle within cells or to a specific intracellular space, for example, targeting to the periplasm or cytoplasm through the addition or removal of a leader sequence that functions in targeting the protein (polypeptide), but is not limited thereto.

Such weakening of the activity of the polypeptide may mean that the activity or concentration (expression level) of the corresponding polypeptide is weakened relative to the activity or concentration of the polypeptide expressed in a wild-type or a microbial strain before modification, or that the amount of a product produced from the corresponding polypeptide is decreased, but is not limited thereto.

The modification of a part or the entirety of the polynucleotide in the microorganism of the present disclosure may be induced by (a) homologous recombination using a vector for chromosomal insertion in a microorganism or genome editing using engineered nuclease (e.g., CRISPR-Cas9), or and/or (b) the treatment with light, such as ultraviolet light and radiation, and/or chemicals, but is not limited thereto.

### Culturing

As used herein, the term "culturing" refers to growing a microorganism in appropriately adjusted environmental conditions. The culturing procedure may be performed according to appropriate media or culture conditions known in the art. Such culturing procedure may be easily adjusted according to the selected microorganism by a person skilled in the art. Specifically, the culturing may be in a batch type, a continuous type, and/or a fed-batch type, but is not limited thereto.

As used herein, the "medium" refers to a mixture comprising, as main ingredients, nutrient materials required for culturing a microorganism, wherein the medium supplies nutrient materials comprising water essential for survival and growth, growth factors, etc. Specifically, as for the media and other culture conditions used for culturing the microorganism of the present disclosure, any medium that is used for usual culture of microorganisms may be used without particular limitation. However, the microorganism of the present disclosure may be cultured under aerobic conditions in a common medium comprising appropriate carbon sources, nitrogen sources, phosphorus sources, inorganic compounds, amino acids, and/or vitamins, etc., while controlling the temperature, pH, etc. For example, a culture medium for microorganisms of the genus *Corynebacterium* may be found in a literature ["Manual of Methods for General Bacteriology" by the American Society for Bacteriology (Washington D.C., USA, 1981)].

In the present disclosure, the carbon source may comprise carbohydrates, such as glucose, saccharose, lactose, fructose, sucrose, maltose, etc.; sugar alcohols, such as mannitol, sorbitol, etc.; organic acids, such as pyruvic acid, lactic acid, citric acid, etc.; and amino acids, such as glutamic acid, methionine, lysine, etc. In addition, natural organic nutrient sources may be used, such as starch hydrolysates, molasses, blackstrap molasses, rice bran, cassava, bagasse, and corn steep liquor, and specifically, carbohydrates, such as glucose and sterile pretreated molasses (i.e., molasses converted to reduced sugars), may be used, and appropriate amounts of other carbon sources may be used without limitation. These carbon sources may be used alone or in a combination of two or more thereof, but are not limited thereto.

As for the nitrogen sources, inorganic nitrogen sources, such as ammonia, ammonium sulfate, ammonium chloride, ammonium acetate, ammonium phosphate, ammonium carbonate, ammonium nitrate, etc.; amino acids, such as glutamic acid, methionine, glutamine, etc.; and organic nitrogen sources, such as peptone, NZ-amine, meat extracts, yeast extracts, malt extracts, corn steep liquor, casein hydrolysates, fish or decomposition products thereof, defatted soybean cake or degradation products thereof, etc. may be used. These nitrogen sources may be used alone or in a combination of two or more thereof, but are not limited thereto.

The phosphate sources may comprise potassium phosphate monobasic, potassium phosphate dibasic, and sodium-comprising salts corresponding thereto. As for inorganic compounds, sodium chloride, calcium chloride, iron chloride, magnesium sulfate, iron sulfate, manganese sulfate, calcium carbonate, etc. may be used, and in addition, amino acids, vitamins, and/or suitable precursors may be comprised. These components or precursors may be added to the medium in a batch or continuous manner. However, the present disclosure is not limited thereto.

Further, pH of the medium may be adjusted by adding compounds, such as ammonium hydroxide, potassium hydroxide, ammonia, phosphoric acid, sulfuric acid, etc., to the medium in an appropriate manner during the culture of the microorganism of the present disclosure. In addition, an anti-foaming agent, such as a fatty acid polyglycol ester, may be used to suppress foam formation during the culture. In addition, oxygen or oxygen-comprising gas may be injected into the medium to maintain the aerobic state of the medium, or no gas may be injected, or nitrogen, hydrogen, or carbon dioxide gas may be injected to maintain the anaerobic or non-aerobic state, but is not limited thereto.

In the culture of the present disclosure, the culture temperature may be maintained at 20°C to 45°C, specifically, at 25°C to 40°C, and the culture may be performed for about 10 hours to 160 hours, but is not limited thereto.

As used herein, the term "culture" refers to a culture liquid obtained by culturing a specific microorganism in a culture medium, a concentrated culture liquid, a dry product of the culture liquid, a culture filtrate, a concentrated culture filtrate, or a dry product of the culture filtrate, and the culture liquid refers to those comprising the specific microorganism, and the culture filtrate refers to those substantially not comprising the specific microorganism (which means that the specific microorganism to be separated by filtration, etc. are substantially excluded, which does not mean that the microorganism is completely excluded from the filtrate.). The formulation of the culture is not limited, but it may be, for example, a liquid, an emulsion, or a solid.

As used herein, the term "fermentation" refers to a process whereby microorganisms break down organic materials using their own enzymes, excluding putrefaction. Fermentation and putrefaction proceed by similar processes. However, when useful substances are produced as a result of decomposition, it is called fermentation, and when bad smells or harmful substances are produced, it is called putrefaction.

In the present disclosure, the method of obtaining the fermentation product from the microorganism is not particularly limited, and the fermentation product may be obtained according to a method commonly used in the art or similar art.

As used herein, the term "fermentation product" comprises not only a fermented material itself, but also all types of materials comprising the fermentation product generated from the microorganism, such as a culture medium of a microorganism in which the microorganism and the culture coexist, a fermentation product obtained by filtering the microorganism from the culture medium, a fermentation product obtained by sterilizing the microorganism from the culture medium and filtering the same, an extract obtained by extracting the fermentation product or the culture medium comprising the same, a dilution obtained by diluting the fermentation product or the extract thereof, a concentrate, a dry product obtained by drying the fermentation product or the extract thereof, a lysate obtained by collecting and disrupting cells of the microorganism, etc.

### Detailed description of the present disclosure

Hereinafter, specific embodiments of the present disclosure will be described in more detail as follows.

An aspect of the present disclosure provides a microorganism with the increased activity of pyruvate dehydrogenase complex dihydrolipoyllysine-residue acetyltransferase, as compared to its endogenous activity.

In one embodiment of the present disclosure, the microorganism of the present disclosure may have an L-amino acid-producing ability.

The increased activity of pyruvate dehydrogenase complex dihydrolipoyllysine-residue acetyltransferase may be defined as the increased L-amino acid-producing ability of the microorganism of the present disclosure, as compared to the L-amino acid-producing ability of a natural wild-type microorganism or unmodified microorganism (e.g., a microorganism expressing a polypeptide having the activity of the wild-type pyruvate dehydrogenase complex dihydrolipoyllysine-residue acetyltransferase (e.g., a polypeptide of SEQ ID NO: 1 or SEQ ID NO: 32) or a microorganism, in which the activity of pyruvate dehydrogenase complex dihydrolipoyllysine-residue acetyltransferase of the present disclosure is not increased, as compared to the endogenous activity, or before the activity is increased), but is not limited thereto.

For example, the activity of pyruvate dehydrogenase complex dihydrolipoyllysine-residue acetyltransferase may be determined by measuring the L-amino acid-producing ability or yield, but is not limited thereto.

As used herein, the term "pyruvate dehydrogenase complex dihydrolipoyllysine-residue acetyltransferase" refers to a component of the pyruvate dehydrogenase complex, which may have dihydrolipoyllysine-residue acetyltransferase activity, i.e., activity of transferring an acetyl group to CoA.

The pyruvate dehydrogenase complex dihydrolipoyllysine-residue acetyltransferase of the present disclosure may be used interchangeably with "AceF". The pyruvate dehydrogenase complex dihydrolipoyllysine-residue acetyltransferase is known in the art, and specifically, may be encoded by *aceF,* but is not limited thereto. The amino acid and polynucleotide sequences of pyruvate dehydrogenase complex dihydrolipoyllysine-residue acetyltransferase may be obtained from known databases, for example, NCBI GenBank, etc., but are not limited thereto.

For example, the pyruvate dehydrogenase complex dihydrolipoyllysine-residue acetyltransferase may comprise an amino acid sequence of SEQ ID NO: 1 or an amino acid sequence having 60% or more homology or identity thereto. However, as long as it has the activity of pyruvate dehydrogenase complex dihydrolipoyllysine-residue acetyltransferase, it is not limited thereto. Specifically, a protein exhibiting efficacy corresponding to that of the pyruvate dehydrogenase complex dihydrolipoyllysine-residue acetyltransferase may be comprised in the pyruvate dehydrogenase complex dihydrolipoyllysine-residue acetyltransferase, even though it comprises a sequence having deletion, modification, substitution, or addition in part of the amino acid sequence of SEQ ID NO: 1. Further, a protein having or comprising the amino acid sequence of SEQ ID NO: 1 or SEQ ID NO: 32 or an amino acid sequence having at least 60%, 70%, 80%, 85%, 90%, 95%, 96%, 97%, 98% or 99% or more homology or identity to the sequence, or consisting of the amino acid sequence or essentially consisting of the amino acid sequence while exhibiting efficacy corresponding to that of the pyruvate dehydrogenase complex dihydrolipoyllysine-residue acetyltransferase may be comprised in the pyruvate dehydrogenase complex dihydrolipoyllysine-residue acetyltransferase. For example, the pyruvate dehydrogenase complex dihydrolipoyllysine-residue acetyltransferase may be a foreign protein or a protein endogenously present in a microorganism of the genus *Corynebacterium* or *Corynebacterium glutamicum,* but is not limited thereto. Specifically, it may be pyruvate dehydrogenase complex dihydrolipoyllysine-residue acetyltransferase consisting of the amino acid sequence of SEQ ID NO: 1 or SEQ ID NO: 32, which is endogenously present in a microorganism of the genus *Corynebacterium* or *Corynebacterium glutamicum,* but is not limited thereto. Examples thereof may comprise NCBI Reference No. WP_011014958.1, WP_060564822.1, WP_074492848.1, etc.

For example, AceF of the present disclosure, pyruvate dehydrogenase complex dihydrolipoyllysine-residue acetyltransferase, may exhibit activity of 2-oxoglutarate dehydrogenase, E2 component, or dihydrolipoamide succinyltransferase. For example, the gene encoding AceF of the present disclosure may be a gene named sucB, but is not limited thereto.

Further, the polynucleotide encoding pyruvate dehydrogenase complex dihydrolipoyllysine-residue acetyltransferase having the amino acid sequence of SEQ ID NO: 1 or an amino acid sequence having 60% or more homology or identity thereto may be prepared, based on codon information known in the art. For example, the protein may be encoded by a polynucleotide having or comprising a sequence of SEQ ID NO: 2 or SEQ ID NO: 33, or a nucleotide sequence having 60% or more, 70% or more, 75% or more, 80% or more, 85% or more, 90% or more, 95% or more, 96% or more, 97% or more, 98% or more, and less than 100% homology or identity to the sequence of SEQ ID NO: 2 or SEQ ID NO: 33, or consisting of or essentially consisting of the nucleotide sequence, but is not limited thereto. Further, the nucleotide sequence of SEQ ID NO: 2 or SEQ ID NO: 33 may be obtained from known databases, for example, NCBI GenBank, etc., but is not limited thereto.

In the present disclosure, a gene comprising a nucleotide sequence represented by a specific sequence number may be used interchangeably with a polynucleotide comprising a nucleotide sequence represented by a specific sequence number.

The polynucleotide of the present disclosure may undergo various modifications in the coding region without changing the amino acid sequence of the pyruvate dehydrogenase complex dihydrolipoyllysine-residue acetyltransferase of the present disclosure in consideration of codon degeneracy or the codons preferred in an organism that is intended to express the pyruvate dehydrogenase complex dihydrolipoyllysine-residue acetyltransferase of the present disclosure. Therefore, it is apparent that a polynucleotide which may be translated to the polypeptide consisting of the amino acid sequence of pyruvate dehydrogenase complex dihydrolipoyllysine-residue acetyltransferase of the present disclosure or a polypeptide having homology or identity thereto due to codon degeneracy may also be comprised in the polynucleotide of the present disclosure. For example, the polynucleotide of the present disclosure may be SEQ ID NO: 2, SEQ ID NO: 33, or a degenerated sequence thereof.

For another example, the polynucleotide of the present disclosure may have or comprise a nucleotide sequence having 60% or more, 70% or more, 75% or more, 80% or more, 85% or more, 90% or more, 95% or more, 96% or more, 97% or more, 98% or more, and less than 100% homology or identity to SEQ ID NO: 2, or may consist of or essentially consist of a nucleotide sequence having 60% or more, 70% or more, 75% or more, 80% or more, 85% or more, 90% or more, 95% or more, 96% or more, 97% or more, 98% or more, and less than 100% homology or identity to SEQ ID NO: 2, but is not limited thereto.

Further, the polynucleotide of the present disclosure may comprise a probe which may be prepared from a known nucleotide sequence, for example, any sequence without limitation as long as it hybridizes with a sequence complementary to the entirety or a part of the polynucleotide sequence of the present disclosure under stringent conditions to encode the pyruvate dehydrogenase complex dihydrolipoyllysine-residue acetyltransferase of the present disclosure.

As used herein, the term "L-amino acid" comprises both proteinaceous and non-proteinaceous amino acids. In the present disclosure, the L-amino acid may comprise glutamate-based products, O-acetylhomoserine, homoserine, and methionine.

As used herein, the term "glutamate-based product" may comprise glutamate as well as L-amino acids that may be biosynthesized using glutamate as a precursor. Examples of the L-amino acids that may be produced through the ornithine cycle using glutamate as a precursor comprise ornithine, citrulline, arginine, and proline, etc. Another example comprises glutamine that may be converted using glutamate as a precursor. In addition, any L-amino acid that may be biosynthesized using glutamate as a precursor may be comprised in the scope of the glutamate-based product of the present disclosure.

In a specific embodiment, the L-amino acid of the present disclosure may be any one or more selected from glutamine, glutamate, ornithine, citrulline, arginine, proline, O-acetylhomoserine, homoserine, and L-methionine. In a specific embodiment, the L-amino acid of the present disclosure may be any one or more selected from ornithine, citrulline, arginine, proline, O-acetylhomoserine, homoserine, and L-methionine.

With respect to the objects of the present disclosure, the microorganism of the present disclosure may comprise any microorganism capable of producing a desired L-amino acid, in which the activity of pyruvate dehydrogenase complex dihydrolipoyllysine-residue acetyltransferase is increased, as compared to its endogenous activity. For example, the microorganism of the present disclosure is characterized in that the activity of pyruvate dehydrogenase complex dihydrolipoyllysine-residue acetyltransferase is increased, as compared to its endogenous activity, and as a result, the L-amino acid-producing ability is increased, and may be a genetically modified microorganism or a recombinant microorganism, but is not limited thereto. Specifically, the recombinant microorganism with the increased L-amino acid-producing ability may be a microorganism with the increased L-amino acid-producing ability, as compared to a natural wild-type microorganism or an unmodified microorganism having the endogenous activity of pyruvate dehydrogenase complex dihydrolipoyllysine-residue acetyltransferase, but is not limited thereto.

For example, the microorganism with the L-amino acid-producing ability may be a prokaryotic or eukaryotic microorganism capable of producing L-amino acids in an organism, and may comprise both a microorganism inherently having the L-amino acid-producing ability or a microorganism prepared by providing the L-amino acid-producing ability for a microorganism inherently not having the L-amino acid-producing ability. The L-amino acid-producing ability may be conferred or enhanced by the increased activity of pyruvate dehydrogenase complex dihydrolipoyllysine-residue acetyltransferase of the present disclosure or species improvement.

For example, the recombinant microorganism having the L-amino acid-producing ability of the present disclosure may comprise any microorganism capable of producing L-amino acids, which is transformed with a vector to have the increased activity of pyruvate dehydrogenase complex dihydrolipoyllysine-residue acetyltransferase of the present disclosure.

For example, the microorganism producing L-amino acids may be a microorganism inherently comprising the protein consisting of the amino acid sequence of SEQ ID NO: 1 or SEQ ID NO: 32, or a protein consisting of an amino acid sequence having at least 60%, 70%, 75%, 80%, 85%, 90%, 95%, 96%, 97%, 98%, 99%, 99.5%, 99.7% or 99.9% or more homology or identity to SEQ ID NO: 1 or SEQ ID NO: 32.

For example, the microorganism producing L-amino acids may be a microorganism inherently comprising a polynucleotide sequence capable of encoding a protein comprising an amino acid sequence having at least 60% homology to SEQ ID NO: 1 or SEQ ID NO: 32, the nucleotide sequence of SEQ ID NO: 2 or SEQ ID NO: 33, or a nucleotide sequence having 60% or more, 70% or more, 75% or more, 80% or more, 85% or more, 90% or more, 95% or more, 96% or more, 97% or more, 98% or more homology or identity to the nucleotide sequence of SEQ ID NO: 2 or SEQ ID NO: 33.

The microorganism of the present disclosure may comprise any microorganism with increased activity of pyruvate dehydrogenase complex dihydrolipoyllysine-residue acetyltransferase, as compared to its endogenous activity, by various known methods.

In one specific embodiment, the microorganism with the increased activity of pyruvate dehydrogenase complex dihydrolipoyllysine-residue acetyltransferase of the present disclosure, as compared to its endogenous activity, may be a microorganism with increased activity by increasing the protein expression through replacement of a sequence regulating the expression of the sequence encoding the pyruvate dehydrogenase complex dihydrolipoyllysine-residue acetyltransferase with another sequence, modification of the nucleotide sequence encoding the pyruvate dehydrogenase complex dihydrolipoyllysine-residue acetyltransferase, or replacement of a start codon, but is not limited thereto. In one specific embodiment, the microorganism with the increased activity of pyruvate dehydrogenase complex dihydrolipoyllysine-residue acetyltransferase of the present disclosure, as compared to its endogenous activity, may be a microorganism with increased activity by increasing the protein expression through replacement of a sequence regulating the expression of the sequence encoding the pyruvate dehydrogenase complex dihydrolipoyllysine-residue acetyltransferase with a sequence having stronger activity than the endogenous expression-regulating sequence, but is not limited thereto.

For example, the microorganism with the increased L-amino acid-producing ability of the present disclosure may be a microorganism with increased L-amino acid-producing ability, as compared to an unmodified microorganism, but is not limited thereto. For example, the unmodified microorganism for comparing whether or not the L-amino acid-producing ability is increased may be ATCC13032 or ATCC13869 strain, but is not limited thereto.

For example, the microorganism with the increased L-amino acid-producing ability may have an increased L-amino acid-producing ability of about 1% or more, specifically, about 1% or more, about 2.5% or more, about 5% or more, as compared to the L-amino acid-producing ability of the parent microorganism (parent strain) before modification or the unmodified microorganism, but the increased amount is not limited thereto as long as the production ability has an increased amount of a + value, as compared to the L-amino acid-producing ability of the parent microorganism (parent strain) before modification or the unmodified microorganism. In another example, the recombinant microorganism with the increased L-amino acid-producing ability may have an increased L-amino acid-producing ability of about 1.01 times or more, 1.02 times or more, 1.03 times or more, 1.04 times or more, 1.05 times or more, 1.06 times or more, 1.07 times or more, 1.08 times or more, 1.09 times or more, or about 1.1 times or more, as compared to the parent microorganism (parent strain) before modification or the unmodified microorganism, but is not limited thereto.

For example, the microorganism with the L-amino acid-producing ability may be either a prokaryotic cell or a eukaryotic cell, specifically, a prokaryotic cell. The prokaryotic cell may comprise, for example, microbial strains of the *Escherichia* sp., *Erwinia* sp., *Serratia* sp., *Providencia* sp., *Corynebacterium* sp., *Pseudomonas* sp., *Leptospira* sp., *Salmonella* sp., *Brevibacteria* sp., *Hypomononas* sp., *Chromobacterium* sp., and *Norcardia* sp., or microbial strains of fungi or yeasts. Specifically, the microorganism may be a microbial strain of the *Escherichia* sp., *Corynebacterium* sp., *Leptospira* sp., and a yeast. More specifically, the microorganism may be a microbial strain of the *Corynebacterium* sp.

With regard to the microorganism according to any one of the aforementioned specific embodiments, the microorganism of the present disclosure may be a microorganism of the *Corynebacterium* sp.

For example of the present disclosure, the microorganism of the present disclosure may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens.* Specifically, the microorganism of the present disclosure may be a microorganism of the *Corynebacterium* sp., more specifically, *Corynebacterium glutamicum,* but is not limited thereto.

The microorganism of the *Corynebacterium* sp. having the L-amino acid-producing ability of the present disclosure may comprise all of a natural wild-type microorganism itself, a microorganism of the *Corynebacterium* sp. that has the enhanced L-amino acid-producing ability by increasing or decreasing the activities of genes related to the L-amino acid production mechanism, or a microorganism of the *Corynebacterium* sp. that has the enhanced L-amino acid-producing ability by introducing or increasing the activity of a foreign gene.

In the microorganism having the L-amino acid-producing ability of the present disclosure, the activity of pyruvate dehydrogenase complex subunit E1 may be further increased, as compared to its endogenous activity.

In the present disclosure, the "pyruvate dehydrogenase complex subunit E1", which is a component of the pyruvate dehydrogenase complex, may be a polypeptide comprising the E1 active site in pyruvate dehydrogenase, which converts pyruvate into acetyl-CoA and CO₂.

The pyruvate dehydrogenase complex subunit E1 of the present disclosure may be used interchangeably with "aceE". The pyruvate dehydrogenase complex subunit E1 is known in the art, and specifically, may be encoded by aceE, but is not limited thereto. The amino acid and polynucleotide sequences of pyruvate dehydrogenase complex subunit E1 may be obtained from known databases, for example, NCBI GenBank, etc., but are not limited thereto.

For example, the pyruvate dehydrogenase complex subunit E1 may comprise an amino acid sequence of SEQ ID NO: 10 or an amino acid sequence having 60% or more homology or identity thereto. However, as long as it has the same activity as pyruvate dehydrogenase complex subunit E1, it is not limited thereto. Specifically, a protein exhibiting efficacy corresponding to that of the pyruvate dehydrogenase complex subunit E1, even though it comprises a sequence having deletion, modification, substitution, or addition in part of the amino acid sequence of SEQ ID NO: 10, may be comprised in the pyruvate dehydrogenase complex subunit E1. Further, a protein having or comprising the amino acid sequence of SEQ ID NO: 10 or an amino acid sequence having at least 60%, 70%, 80%, 85%, 90%, 95%, 96%, 97%, 98% or 99% or more homology or identity to the sequence, or consisting of the amino acid sequence or essentially consisting of the amino acid sequence while exhibiting efficacy corresponding to that of the pyruvate dehydrogenase complex subunit E1 may be comprised in the pyruvate dehydrogenase complex subunit E1. For example, the pyruvate dehydrogenase complex subunit E1 may be a foreign protein or a protein endogenously present in a microorganism of the genus *Corynebacterium* or *Corynebacterium glutamicum,* but is not limited thereto. Specifically, it may be pyruvate dehydrogenase complex subunit E1 consisting of the amino acid sequence of SEQ ID NO: 10, which is endogenously present in a microorganism of the genus *Corynebacterium* or *Corynebacterium glutamicum,* but is not limited thereto. Examples thereof may comprise NCBI Reference No. WP_011014985.1, etc.

Further, the polynucleotide encoding pyruvate dehydrogenase complex subunit E1 having the amino acid sequence of SEQ ID NO: 10 or an amino acid sequence having 60% or more homology or identity thereto may be prepared, based on codon information known in the art. For example, the protein may be encoded by a polynucleotide having or comprising a sequence of SEQ ID NO: 11 or SEQ ID NO: 39, or a nucleotide sequence having 60% or more, 70% or more, 75% or more, 80% or more, 85% or more, 90% or more, 95% or more, 96% or more, 97% or more, 98% or more, and less than 100% homology or identity to the sequence of SEQ ID NO: 11 or SEQ ID NO: 39, or consisting of or essentially consisting of the nucleotide sequence, but is not limited thereto. Further, the nucleotide sequence of SEQ ID NO: 11 or SEQ ID NO: 39 may be obtained from known databases, for example, NCBI GenBank, etc., but is not limited thereto.

The microorganism having the L-amino acid-producing ability of the present disclosure may further comprise a modification that further increases the desired L-amino acid-producing ability.

For example, the microorganism of the present disclosure may be a microorganism in which the activity of arginine transcriptional repressor (ArgR) is weakened. For example thereof, the microorganism of the present disclosure may be a microorganism in which the entirety or a part of the argR gene is additionally deleted. For example, the microorganism of the present disclosure may be a microorganism in which methionine at position 54 of acetylglutamate kinase (ArgB) is substituted with valine.

For example, the microorganism of the present disclosure may be a microorganism in which the activity of N-acetyl-gamma-glutamyl-phosphate reductase (hereinafter, referred to as ArgC) is increased, as compared to its endogenous activity.

For example, the microorganism of the present disclosure may be a microorganism in which the activity of argininosuccinate synthetase (ArgG) is weakened. For example thereof, the microorganism of the present disclosure may be a microorganism in which the entirety or a part of the argG gene is additionally deleted.

For example, the microorganism of the present disclosure may be a microorganism in which the activity of L-lysine exporter LysE is weakened. For example thereof, the microorganism of the present disclosure may be a microorganism in which the entirety or a part of the lysE gene encoding L-lysine exporter is deleted.

For example, the microorganism of the present disclosure may be a microorganism in which the activity of carbamoyl phosphate synthase is weakened. For example thereof, the microorganism of the present disclosure may be a microorganism in which the activity of carbamoyl phosphate synthase small subunit (CarA) and/or carbamoyl phosphate synthase large subunit (CarB) are/is weakened.

For example, the microorganism of the present disclosure may be a microorganism in which the activity of ornithine carbamoyltransferase F (ArgF) is weakened. For example thereof, the microorganism of the present disclosure may be a microorganism in which the entirety or a part of the argF gene is deleted.

For example, the microorganism of the present disclosure may be a microorganism into which a foreign ornithine export protein is introduced. Examples thereof may be a microorganism into which a membrane protein derived from a microorganism of the *Shewanella* sp. is introduced, for example, a microorganism into which a membrane protein derived from *Shewanella oneidensis* is introduced.

For example, the microorganism of the present disclosure may comprise a modification of inner membrane protein YjeH. In this regard, the content of WO2021-125896 A1 may be incorporated as a reference.

For example, the microorganism of the present disclosure may be a microorganism in which the activity of O-acetyl-homoserine transferase (MetX) is increased, as compared to its endogenous activity.

For example, the microorganism of the present disclosure may comprise a modification in which the expression of aspartokinase (LysC) is enhanced and/or feedback inhibition is released. In this regard, the content of US 10662450 B2 may be incorporated as a reference.

For example, the microorganism of the present disclosure may be a microorganism in which the activity of cystathionine gamma-synthase is weakened. Examples thereof may be a microorganism in which the activity of the metB gene encoding cystathionine gamma-synthase is weakened. For example, the microorganism may be a microorganism in which the entirety or a part of the metB gene is deleted.

For example, the microorganism of the present disclosure may be a microorganism in which the activity of O-acetylhomoserine (thiol)-lyase is weakened. For example thereof, the microorganism of the present disclosure may be a microorganism in which the activity of the metY gene encoding O-acetylhomoserine (thiol)-lyase is weakened. For example, the microorganism may be a microorganism in which the entirety or a part of the metY gene is deleted.

For example, the microorganism of the present disclosure may be a microorganism in which the activity of an endogenous protein Ncgl0616 is weakened. Examples thereof may be a microorganism in which a part or the entirety of a gene encoding Ncgl0616 is deleted.

Still another aspect of the present disclosure provides a method of producing an L-amino acid, the method comprising the step of culturing, in a medium, the microorganism with increased activity of pyruvate dehydrogenase complex dihydrolipoyllysine-residue acetyltransferase of the present disclosure, as compared to its endogenous activity.

With regard to the method of the present disclosure, the culturing of the microorganism may be performed using any culturing conditions and culturing method known in the art. Such culturing procedure may be easily adjusted for use by a person skilled in the art according to the selected strain.

The L-amino acid produced by culturing of the present disclosure may be released into the medium or may remain in cells.

In one specific embodiment, the method of producing an L-amino acid of the present disclosure may further comprise the steps of preparing the microorganism of the present disclosure, preparing a medium for culturing the strain, or a combination of these steps (regardless of the order, in any order), for example, before the culturing step.

The method of producing an L-amino acid of the present disclosure may further comprise the step of recovering a desired substance, specifically, the L-amino acid from the cultured microorganism, a culture of the microorganism, a fermentation product of the microorganism, or the culture medium. The recovering step may be further comprised after the culturing step.

The recovering may be collecting a desired L-amino acid by using an appropriate method known in the art according to the method of culturing the microorganism of the present disclosure, for example, a batch, continuous, or fed-batch type culture. For example, centrifugation, filtration, treatment with a crystallized protein precipitating agent (salting-out), extraction, sonication, ultrafiltration, dialysis, various types of chromatography, such as molecular sieve chromatography (gel filtration), adsorption chromatography, ion exchange chromatography, affinity chromatography, HPLC, and a combination of these methods may be used, and a desired substance, specifically, L-amino acid may be recovered from the medium or microorganism by using an appropriate method known in the art.

In addition, the method of producing an L-amino acid of the present disclosure may further comprise a purification step. The purification may be performed by using an appropriate method known in the art. In an exemplary embodiment, when the method of producing an L-amino acid of the present disclosure comprises both the recovering step and the purification step, the recovering step and the purification step may be performed continuously or discontinuously regardless of the order, or may be performed simultaneously or integrated into one step, but is not limited thereto.

With regard to the method of the present disclosure, the increase of the pyruvate dehydrogenase complex dihydrolipoyllysine-residue acetyltransferase activity and L-amino acid, etc. are as described in other aspects.

Still another aspect of the present disclosure provides a composition for producing an L-amino acid, the composition comprising the microorganism with increased activity of pyruvate dehydrogenase complex dihydrolipoyllysine-residue acetyltransferase of the present disclosure, as compared to its endogenous activity, the culture of the microorganism, the fermentation product of the microorganism, or a combination of two or more thereof.

The composition of the present disclosure may further comprise any appropriate excipient that is usually used in the composition for producing L-amino acids, and examples of the excipient may comprise a preserving agent, a wetting agent, a dispersing agent, a suspending agent, a buffer, a stabilizing agent, an isotonic agent, etc., but are not limited thereto.

In a specific embodiment, each component present in the composition of the present disclosure may be comprised in a microbially effective amount, or in an amount that may be appropriately present in the composition for production.

With regard to the composition of the present disclosure, the increase of the pyruvate dehydrogenase complex dihydrolipoyllysine-residue acetyltransferase activity and L-amino acid, etc. are as described in other aspects.

Still another aspect of the present disclosure provides use of the microorganism with increased activity of pyruvate dehydrogenase complex dihydrolipoyllysine-residue acetyltransferase of the present disclosure, as compared to its endogenous activity, in producing an L-amino acid.

With regard to the use of the present disclosure, the increase of the pyruvate dehydrogenase complex dihydrolipoyllysine-residue acetyltransferase activity and L-amino acid, etc. are as described in other aspects.

Still another aspect of the present disclosure provides a method of preparing an L-amino acid-producing microorganism, the method comprising modifying a microorganism such that the microorganism has increased activity of pyruvate dehydrogenase complex dihydrolipoyllysine-residue acetyltransferase of the present disclosure, as compared to its endogenous activity.

With regard to the method of preparing the microorganism of the present disclosure, the increase of the pyruvate dehydrogenase complex dihydrolipoyllysine-residue acetyltransferase activity and L-amino acid, etc. are as described in other aspects.

### [Mode for Carrying Out the Invention]

Hereinafter, the present disclosure will be described in more detail with reference to Examples and Experimental Examples. However, these Examples and Experimental Examples are only for illustrating the present disclosure, and the scope of the present disclosure is not intended to be limited by these Examples and Experimental Examples.

### Example 1. Construction of plasmid for enhancing activity of pyruvate dehydrogenase complex dihydrolipoyllysine-residue acetyltransferase

### Example 1-1: Construction of plasmid for replacing promoter

To determine the effectiveness of a gene (NCBI accession number BBD29_RS10495, hereinafter referred to as aceF) encoding *Corynebacterium glutamicum* pyruvate dehydrogenase complex dihydrolipoyllysine-residue acetyltransferase (NCBI accession number WP_060564822.1), a vector for enhancing the gene number BBD29_RS10495 (SEQ ID NO: 2) derived from *Corynebacterium glutamicum* ATCC13869 was constructed. In detail, to construct a vector for enhancing pyruvate dehydrogenase complex dihydrolipoyllysine-residue acetyltransferase, a plasmid for enhancing aceF activity was constructed by replacing the wild-type promoter of the aceF gene with the Pcj7 promoter (US 7662943 B2) (SEQ ID NO: 3), which is known as a strong promoter.

The upstream and downstream regions of the aceF gene were obtained. In detail, in order to prepare a strain into which aceF having the Pcj7 promoter was introduced, the chromosomal DNA of *Corynebacterium glutamicum* ATCC13869 was used as a template, and primers of SEQ ID NO: 4 and SEQ ID NO: 5 for the upstream region of the aceF gene and primers of SEQ ID NO: 6 and SEQ ID NO: 7 for the downstream region of the aceF gene were used to perform PCR, respectively. In addition, the Pcj7 promoter fragment was obtained using pDCM2-Pcj7 as a template and SEQ ID NO: 8 and SEQ ID NO: 9. The primer sequences used to perform each PCR are as shown in Table 1 below.

**[Table 1]**

| SEQ ID NO. | Name | Sequence |
|---|---|---|
| 4 | aceF-5'-F | GTGAATTCGAGCTCGGTACCCCGAGCTTTTTGTTCC TCACGC |
| 5 | aceF -5'-R | TAGTAGCGCTGGGATGTTTCTTATTTTAAGACTCCTC GCAAG |
| 6 | aceF -3'-F | ACCCAACGAAAGGAAACACTCATGGCGTTCTCCGT AGAGATG |
| 7 | aceF -3'-R | GGTCGACTCTAGAGGATCCCCCGTCTACTTCGACG GTGTCGC |
| 8 | Pcj7-F | AGAAACATCCCAGCGCTACTAATAGGGAGCGTTG |
| 9 | Pci7-R | ATGGGATACGTACCCAACGAAAGGAAACACTC |

PfuUltra^{™} high-fidelity DNA polymerase (Stratagene) was used as a polymerase for PCR reaction, and the PCR conditions consisted of denaturation at 95°C for 30 sec; annealing at 55°C for 30 sec; and polymerization at 72°C for 1 min. Denaturation, annealing, and polymerization of these conditions were repeated for 28 cycles. As a result, a 318-bp DNA fragment of the Pcj7 promoter region, a 526-bp upstream DNA fragment and a 529-bp downstream DNA fragment of *Corynebacterium glutamicum* ATCC13869 aceF were obtained, respectively. PCR was performed using the amplified promoter and DNA fragments as templates, and primers of SEQ ID NO: 4 and SEQ ID NO: 7. The PCR conditions consisted of denaturation at 95°C for 5 min, followed by 28 cycles of denaturation at 95°C for 30 sec; annealing at 55°C for 30 sec; and polymerization at 72°C for 2 min, and then polymerization at 72°C for 5 min. The two fragments obtained above were subjected to DNA purification, and fusion cloning was performed according to the provided manual using In-Fusion^{®} HD cloning kit (Clontech) and Smal restriction enzyme-treated pDCM2 vector (WO2021-187781 A1) to obtain a plasmid. The resulting vector was named pDCM2-Pcj7_aceF.

### Example 1-2: Construction of plasmid for enhancing activity of pyruvate dehydrogenase complex subunit E1

A vector for enhancing the activity of pyruvate dehydrogenase complex subunit E1 (NCBI accession number_BBD29_RS10685, hereinafter referred to as aceE) was constructed. In detail, to prepare a vector for enhancing pyruvate dehydrogenase complex subunit E1 (BBD29_RS10685, SEQ ID NO: 11) of SEQ ID NO: 10 (NCBI Reference No. WP_011014985.1), a plasmid for enhancing aceE activity was constructed by replacing the wild-type promoter of the aceE gene with the Pcj7 promoter (US 7662943 B2) (SEQ ID NO: 3), which is known as a strong promoter.

The upstream and downstream regions of the aceE gene were obtained. In detail, in order to prepare a strain into which aceE having the Pcj7 promoter was introduced, the chromosomal DNA of *Corynebacterium glutamicum* ATCC13869 was used as a template, and primers of SEQ ID NO: 12 and SEQ ID NO: 13 for the upstream region of the aceE gene and primers of SEQ ID NO: 14 and SEQ ID NO: 15 for the downstream region of the aceE gene were used to perform PCR, respectively. In addition, the Pcj7 promoter fragment was obtained using pDCM2-Pcj7 as a template and SEQ ID NO: 8 and SEQ ID NO: 9. The primer sequences used to perform each PCR are as shown in Table 2 below.

**[Table 2]**

| SEQ ID NO. | Name | Sequence |
|---|---|---|
| 12 | aceE -5'-F | GTGAATTCGAGCTCGGTACCCCACTGGCCAGCG ATTAACTTT |
| 13 | aceE -5'-R | TAGTAGCGCTGGGATGTTTCTTTCCACACCTCCT GTTGGAAT |
| 14 | aceE -3'-F | CCAACGAAAGGAAACACTCATGGCCGATCAAGC AAAACTT |
| 15 | aceE -3'-R | GGTCGACTCTAGAGGATCCCCCCTGGAAGAAGA TCTGGTCGC |

PfuUltra^{™} high-fidelity DNA polymerase (Stratagene) was used as a polymerase for PCR reaction, and the PCR conditions consisted of denaturation at 95°C for 30 sec; annealing at 55°C for 30 sec; and polymerization at 72°C for 1 min. Denaturation, annealing, and polymerization of these conditions were repeated for 28 cycles. As a result, a 318-bp DNA fragment of the Pcj7 promoter region, a 526-bp upstream DNA fragment and a 527-bp downstream DNA fragment of *Corynebacterium glutamicum* ATCC13869 aceE were obtained, respectively. PCR was performed using the amplified promoter and DNA fragments as templates, and primers of SEQ ID NO: 12 and SEQ ID NO: 15. The PCR conditions consisted of denaturation at 95°C for 5 min, followed by 28 cycles of denaturation at 95°C for 30 sec; annealing at 55°C for 30 sec; and polymerization at 72°C for 2 min, and then polymerization at 72°C for 5 min. The two fragments obtained above were subjected to DNA purification, and fusion cloning was performed according to the provided manual using In-Fusion^{®} HD cloning kit (Clontech) and Smal restriction enzyme-treated pDCM2 vector (WO2021-187781 A1) to obtain a plasmid. The resulting vector was named pDCM2-Pcj7_aceE.

### Example 2: Preparation of microorganism with enhanced activity of pyruvate dehydrogenase complex dihydrolipoyllysine-residue acetyltransferase and Evaluation of L-amino acid-producing ability

### Example 2-1. Preparation of Corynebacterium glutamicum CJR2 strain

*Corynebacterium glutamicum* strain CJR2 was prepared in order to evaluate L-arginine, citrulline, and ornithine-producing abilities.

This is to introduce mutations (ΔargR, argB(M54V)) into the wild-type *Corynebacterium glutamicum* ATCC13869 (Ikeda, Masato et al., Applied and environmental microbiology 75(6)1635-41, 2009).

First, a vector for introducing argR deletion and argB(M54V) mutation was constructed. PCR was performed using genomic DNA of *Corynebacterium glutamicum* ATCC13869 as a template, and primer pairs in Table 3 (SEQ ID NOS: 16 and 17, SEQ ID NOS: 18 and 19), and overlapping PCR was performed using a pair of primers of SEQ ID NOS: 16 and 19 to obtain homologous recombinant fragments having the argR deletion mutation sequence. In the same manner, to prepare a homologous recombinant fragment having argB(M54V) mutation, PCR was performed using primer pairs (SEQ ID NOS: 20 and 21, SEQ ID NOS: 22 and 23) in Table 3 and overlapping PCR was performed using SEQ ID NOS: 20 and 23. PCR reactions were performed with 30 cycles of denaturation at 95°C for 30 sec; annealing at 55°C for 30 sec; and extension at 72°C for 2 min. The linearized pDCM2 vector and each homologous recombinant fragment were subjected to fusion cloning in the same manner as Example 2. The constructed vectors (recombinant plasmids) were named pDCM2-ΔargR and pDCM2-argB(M54V), respectively.

Subsequently, the argR deletion mutation was introduced into the wild-type *Corynebacterium glutamicum* ATCC13869. Transformation was performed using the prepared pDCM2-ΔargR plasmid by an electric pulse method (van der Rest et al., Appl Microbiol Biotechnol 52:541-545, 1999). Then, the secondary recombination was performed on a solid plate medium comprising 4% sucrose, and the transformant in which the secondary recombination was completed was subjected to PCR using a pair of primers (SEQ ID NOS: 16 and 19) to confirm that the deletion mutation was introduced into the argR gene on the chromosome. In this regard, the PCR reaction was performed by repeating 30 cycles of denaturation at 95°C for 30 sec; annealing at 55°C for 30 sec; and extension at 72°C for 2 min. The above transformant was named CJR1.

### <Complex plate medium (pH 7.0)>

Glucose 10 g, Peptone 10 g, Beef extract 5 g, Yeast extract 5 g, Brain heart infusion 18.5 g, NaCl 2.5 g, urea 2 g, sorbitol 91 g, agar 20 g (based on 1 liter of distilled water)

Next, the argB (M54V) mutation was introduced into *Corynebacterium glutamicum* CJR1 in the same manner as above. The constructed pDCM2-argB (M54V) plasmid was used, and the transformant in which the secondary recombination was completed was subjected to PCR using a pair of primers (SEQ ID NOS: 20 and 23), confirming that the M54V mutation was introduced into the argB gene on the chromosome, and the transformant was named CJR2.

**[Table 3]**

| SEQ ID NO. | Name | Sequence |
|---|---|---|
| SEQ ID NO: 16 | argR-5'-F | tgaattcgagctcggtaccccactggtgaactccttgtcc |
| SEQ ID NO: 17 | argR-5'-R | ttgaactaggggcgctttaaaagttttccggtgttgacgg |
| SEQ ID NO: 18 | argR-3'-F | ccgtcaacaccggaaaacttttaaagcgcccctagttcaa |
| SEQ ID NO: 19 | argR-3'-R | gtcgactctagaggatcccccgttgaactgcttgccagcc |
| SEQ ID NO: 20 | argB-5'-F | tgaattcgagctcggtaccctgcggctcgcacggttgctc |
| SEQ ID NO: 21 | arqB-5'-R | acggtgcgcaagaagaccacgtcggcagcaaaagcagcct |
| SEQ ID NO: 22 | argB-3'-F | ggetgcttttgctgccgacgtggtcttcttgcgcaccgtg |
| SEQ ID NO: 23 | argB-3'-R | gtcgactctagaggatccccctcttatcaggccaatcggt |

### Example 2-2. Preparation of Corynebacterium glutamicum CJR100 strain

Based on the CJR2 strain prepared in Example 2-1, it was intended to prepare a CJR100 strain in which the N-acetyl-gamma-glutamyl-phosphate reductase (hereinafter, referred to as argC) gene was enhanced.

In order to enhance the activity of argC (SEQ ID NO: 25_NCBI accession number BBD29_RS07530), which is an N-acetyl-gamma-glutamyl-phosphate reductase, a plasmid for enhancing the argC activity was prepared by replacing the wild-type promoter of the argC gene with Po2 using the Po2 promoter (US 10273491 B2), which is known as a strong promoter. The upstream and downstream regions of the argC gene were obtained. In detail, to prepare a strain into which argC with the Po2 promoter was introduced, PCR was performed using chromosomal DNA of *Corynebacterium glutamicum* ATCC13869 as a template and primers of SEQ ID NO: 26 and SEQ ID NO: 27 for amplifying the upstream region of the argC gene, and primers of SEQ ID NO: 28 and SEQ ID NO: 29 for amplifying the downstream region of the argC gene, respectively. In addition, the Po2 promoter fragment was obtained using pDCM2-Po2 as a template and SEQ ID NO: 30 and SEQ ID NO: 31. PfuUltra^{™} high-fidelity DNA polymerase (Stratagene) was used as polymerase for the PCR reaction, and the PCR conditions consisted of denaturation at 95°C for 30 sec; annealing at 55°C for 30 sec; and polymerization at 72°C for 1 min. Denaturation, annealing, and polymerization of these conditions were repeated for 28 cycles. As a result, an 86-bp DNA fragment of the Po2 promoter region, a 610-bp upstream DNA fragment and a 1086-bp downstream DNA fragment of *Corynebacterium glutamicum* ATCC13869 argC were obtained, respectively. PCR was performed using the amplified promoter and DNA fragments as templates and primers of SEQ ID NO: 26 and SEQ ID NO: 29. The PCR conditions consisted of denaturation at 95°C for 5 min, followed by 28 cycles of denaturation at 95°C for 30 sec; annealing at 55°C for 30 sec; and polymerization at 72°C for 2 min, and then polymerization at 72°C for 5 min. The two fragments obtained above were subjected to DNA purification, and linked to Smal restriction enzyme-treated pDCM2 plasmid by fusion cloning using In-Fusion^{®} HD cloning kit (Clontech). The resulting vector was named pDCM2-Po2-argC.

Subsequently, the CJR2 strain prepared in Example 2-1 was transformed with the pDCM2-Po2-argC plasmid constructed above by an electric pulse method (van der Rest et al., Appl Microbiol Biotechnol 52:541-545, 1999). Subsequently, secondary recombination was performed on a solid plate medium comprising 4% sucrose, and the transformant in which the secondary recombination was completed was subjected to PCR using a pair of primers (SEQ ID NOS: 26 and 31) to confirm that the argC gene on the chromosome was enhanced with the Po2 promoter. In this regard, the PCR reaction was performed by repeating 30 cycles of denaturation at 95°C for 30 sec; annealing at 55°C for 30 sec; and extension at 72°C for 2 min. The above transformant was named CJR100.

### <Complex plate medium (pH 7.0)>

Glucose 10 g, Peptone 10 g, Beef extract 5 g, Yeast extract 5 g, Brain heart infusion 18.5 g, NaCl 2.5 g, urea 2 g, sorbitol 91 g, agar 20 g (based on 1 liter of distilled water)

The sequences and detailed information of the primers used in this Example are listed in Table 4.

**[Table 4]**

| SEQ ID NO. | Name | Sequence |
|---|---|---|
| SEQ ID NO: 26 | argC-5'-F | GTGAATTCGAGCTCGGTACCCGCCCCGAAAAG CCGTTAAAAG |
| SEQ ID NO: 27 | argC-5'-R | tgccaaaattcacgattattgCTCGAGTCTAGAGACGGGT TA |
| SEQ ID NO: 28 | argC-3'-F | ttattggaggagatcaaaacaATGACAATCAAGGTTGCA ATC |
| SEQ ID NO: 29 | argC-3'-R | CAGGTCGGCGTCGCACCTTAAGGGGATCCTCT AGAGTCGACC |
| SEQ ID NO: 30 | Po2-F | caataatcgtgaattttggca |
| SEQ ID NO: 31 | Po2-R | tgttttgatctcctccaataa |

### Example 2-3. Preparation of strain with enhanced activities of pyruvate dehydrogenase complex dihydrolipoyllysine-residue acetyltransferase and pyruvate dehydrogenase complex subunit E1 and Evaluation of arginine, citrulline, and ornithine-producing abilities

Based on the CJR100 strain prepared in Example 2-2, it was transformed with the pDCM2-Pcj7_aceF plasmid constructed in Example 1 by an electric pulse method (van der Rest et al., Appl Microbiol Biotechnol 52:541-545, 1999). Subsequently, secondary recombination was performed on a solid plate medium comprising 4% sucrose, and the transformant in which the secondary recombination was completed was subjected to PCR using a pair of primers (SEQ ID NOS: 4 and 9) to confirm that pyruvate dehydrogenase complex dihydrolipoyllysine-residue acetyltransferase was enhanced with the Pcj7 promoter. In this regard, the PCR reaction was performed by repeating 30 cycles of denaturation at 95°C for 30 sec; annealing at 55°C for 30 sec; and extension at 72°C for 2 min. The above transformant was named CJR101 (CJR100-Pcj7_aceF).

Further, based on the CJR101 strain in which aceF was enhanced, it was transformed with the pDCM2-Pcj7_aceE plasmid constructed in Example 1-2 by the electric pulse method (van der Rest et al., Appl Microbiol Biotechnol 52:541-545, 1999). Subsequently, secondary recombination was performed on a solid plate medium comprising 4% sucrose, and the transformant in which the secondary recombination was completed was subjected to PCR using a pair of primers (SEQ ID NOS: 12 and 19) to confirm that pyruvate dehydrogenase complex subunit E1 on the chromosome was enhanced with the Pcj7 promoter. In this regard, the PCR reaction was performed by repeating 30 cycles of denaturation at 95°C for 30 sec; annealing at 55°C for 30 sec; and extension at 72°C for 2 min. The above transformant was named CJR103 (CJR101-Pcj7_aceE).

The parent strain *Corynebacterium glutamicum* CJR2 and the strains CJR101, CJR102, and CJR103 prepared in Example 2-3 were inoculated in a 250 ml corner-baffled flask comprising 25 ml of the production medium below, respectively and cultured at 30°C for 44 hours with shaking at 200 rpm. The composition of the production medium is as follows.

### <Complex plate medium (pH 7.0)>

Glucose 10 g, Peptone 10 g, Beef extract 5 g, Yeast extract 5 g, Brain heart infusion 18.5 g, NaCl 2.5 g, urea 2 g, sorbitol 91 g, agar 20 g (based on 1 liter of distilled water)

### <Production medium (pH 7.2)>

Sucrose 50 g, Ammonium sulfate 57 g, Magnesium sulfate heptahydrate 2 g, Beet molasses 5 g, Calcium chloride 1 mg, Cobalt chloride 1 mg, Potassium phosphate monobasic 2 g, Biotin 0.01 mg, Thiamine-HCl 0.1 mg, Calcium pantothenate 2 mg, Nicotinamide 3 mg, Ferrous sulfate 10 mg, Manganese sulfate 10 mg, Zinc sulfate 0.02 mg, Copper sulfate 0.5 mg, Calcium carbonate 30 g (based on 1 liter of distilled water)

After the culturing was completed, the productivity (concentration) of L-arginine was analyzed using HPLC (Waters 2478), and the analyzed L-arginine and L-citrulline concentrations and the improvement of L-arginine yield are shown in Table 5 below.

**[Table 5]**

| Arginine, citrulline, and ornithine productivity | | | | | |
|---|---|---|---|---|---|
| Strain | | L-arginine (q/I) | | L-citrulline (g/l) | ornithine (g/l) |
| | | 3BT mean | Improvement | 3BT mean | 3BT mean |
| Control | CJR2 | 5.1 | - | 1.0 | 0.1 |
| Control | CJR100 | 5.9 | - | 1.0 | 0.2 |
| AceF enhancement | CJR101 | 6.37 | 108% | 1.3 | 0.4 |
| AceF + AceE enhancement | CJR103 | 7.00 | 110% | 1.7 | 0.7 |

As a result, it was confirmed that the L-arginine-producing ability of the strain CJR101, in which aceF was enhanced with the Pcj7 promoter, improved by 8% to 10%, as compared to the parent strain CJR100, in which aceF was not enhanced. It was also confirmed that the L-arginine-producing ability of the strain CJR103, in which aceE was enhanced in the CJR101 strain with the aceF enhancement, improved by 110%, as compared to the parent strain CJR101, and improved by 118%, as compared to CJR100.

It was also confirmed that the L-citrulline and L-ornithine-producing abilities of the CJR101 and CJR103 strains improved, as compared to control strains (CJR2, CJR100). These results indicate that the enhancement of pyruvate dehydrogenase complex dihydrolipoyllysine-residue acetyltransferase was effective for L-arginine production, and the additional enhancement of pyruvate dehydrogenase complex subunit E1 was effective for arginine production in the L-arginine-producing strains of the genus *Corynebacterium.* In addition, it was found that the enhancement is also effective for the production of not only arginine but also citrulline and ornithine.

### Example 3: Preparation of citrulline-producing strain with enhanced activities of pyruvate dehydrogenase complex dihydrolipoyllysine-residue acetyltransferase and pyruvate dehydrogenase complex subunit E1 and Evaluation of citrulline-producing ability

### Example 3-1. Preparation of citrulline-producing strain

A strain to be used as a control group for the evaluation of the citrulline-producing ability was prepared.

A vector for substituting a stop codon for glutamic acid at position 47 of a protein sequence of argR (ANU33619.1) was constructed. The genome of wild-type *C*. *glutamicum* ATCC 13869 was used as a template to amplify the homologous recombinant A arm using a pair of primers of SEQ ID NOS: 40 and 41, and the homologous recombinant B arm using a pair of primers of SEQ ID NOS: 42 and 43. Thereafter, a plasmid was obtained in the same manner as Example 1, and this plasmid was named pDCM2-argR(E47*).

In order to prepare a microorganism in which the L-citrulline-producing ability was further improved, a vector for substituting a stop codon for phenylalanine at position 68 of a protein sequence of argG (ANU33620.1) was constructed. The genome of *C*. *glutamicum* ATCC13869 was used as a template to amplify the homologous recombinant A arm using a pair of primers of SEQ ID NOS: 44 and 45, and the homologous recombinant B arm using a pair of primers of SEQ ID NOS: 46 and 47. Thereafter, a plasmid was obtained using the method described above, and this plasmid was named pDCM2- argG(F68*).

The primer sequences used here are as shown in Table 6 below.

**[Table 6]**

| SEQ ID NO. | Sequence (5' -> 3') |
|---|---|
| 40 | CGGTACCCGGGGATCCCTCGTGCGGAAT TCGTGGAG |
| 41 | ATCCAGCAGCAATTCAGACA |
| 42 | CTGAATTGCTGCTGGATTAAGGCATCGATA TCACCCA |
| 43 | ATGCCTGCAGGTCGACCCTTCATTTTAAGT TCCTTG |
| 44 | CGGTACCCGGGGATCCTTCATCGATAGGG TGGG |
| 45 | GTACTCCTCAGCTTACTCATCCTTTGCATC AACA |
| 46 | AGTAAGCTGAGGAGTACTGCCTGCCAACC ATCAA |
| 47 | ATGCCTGCAGGTCGACCGACTGGCTTGC CACCCT |

The wild-type *C*. *glutamicum* ATCC 13869 was transformed using the constructed pDCM2-argR(E47*) vector by electroporation (Appl. Microbiol.Biotechnol. (1999) 52:541-545), and then through a secondary crossover process, a strain was obtained in which the 139^{th} nucleotide sequence of argR was substituted from guanine (g) to thymine (t), and the 47^{th} protein sequence was substituted with a stop codon. PCR and nucleotide sequence analysis were performed using a pair of primers of SEQ ID NOS: 40 and 43 capable of amplifying the adjacent region comprising the position where the corresponding gene was inserted, and the corresponding genetic manipulation was confirmed. The microorganism thus obtained was named C. gl::argR*.

To prepare a microorganism, in which citrulline production was further enhanced in C. gl::argR*, a microorganism was obtained using the pDCM2-argG(F68*) vector in the same manner as described above. PCR and nucleotide sequence analysis were performed using a pair of primers of SEQ ID NOS: 44 and 47 capable of amplifying the adjacent region comprising the position where the corresponding gene was inserted, and the corresponding genetic manipulation was confirmed. The microorganism thus obtained was named C. gl::argR*_argG*.

### Example 3-2. Preparation of citrulline-producing strain with enhanced activities of pyruvate dehydrogenase complex dihydrolipoyllysine-residue acetyltransferase and pyruvate dehydrogenase complex subunit E1

In order to examine whether or not the effect of increasing citrulline production was present in the strain with enhanced activity of pyruvate dehydrogenase complex in *Corynebacterium glutamicum* strain having the L-citrulline-producing ability, a strain was prepared by enhancing the aceF activity in C. gl::argR*_argG*, which is an L-citrulline-producing strain prepared in Example 3-1.

In detail, the pDCM2-Pcj7_aceF plasmid which is a vector prepared in Example 1 was used to transform C. gl::argR*_argG*, which is an L-citrulline-producing strain, using the electric pulse method (van der Rest et al., Appl Microbiol Biotechnol 52:541-545, 1999). The transformant was named C. gl::argR*_argG*-Pcj7_aceF.

Based on the C. gl::argR*_argG*-Pcj7_aceF strain, a strain was prepared in which the aceE activity was further enhanced. In detail, the pDCM2-Pcj7_aceE plasmid which is a vector prepared in Example 1-2 was used to transform an L-citrulline-producing strain C. gl::argR*_argG*_Pcj7_aceF by the electric pulse method (van der Rest et al., Appl Microbiol Biotechnol 52:541-545, 1999). The transformant was named C. gl::argR*_argG*-Pcj7_aceF-Pcj7_aceE.

### Example 3-3. Evaluation of citrulline-producing ability of strain with enhanced activities of pyruvate dehydrogenase complex dihydrolipoyllysine-residue acetyltransferase and pyruvate dehydrogenase complex subunit E1

To compare the citrulline-producing ability between citrulline-producing strains, *Corynebacterium glutamicum* C. gl::argR*_argG* and C. gl::argR*_argG*-Pcj7_-aceF, C. gl::argR*_argG*-Pcj7_aceF-Pcj7_aceE, a flask test was performed. First, each strain was inoculated into a 250 ml corner-baffled flask comprising 25 ml of a seed medium, and cultured at 30°C for 20 hours with shaking at 200 rpm. 1 ml of the seed culture was inoculated into a 250 ml corner-baffled flask comprising 24 ml of a production medium, and cultured at 33°C for 42 hours with shaking at 200 rpm. After the culturing was completed, the production amount of L-citrulline was measured by HPLC, and the results are shown in Table 7 below.

### <Seed medium (pH 7.0)>

Glucose 20 g, Peptone 10 g, Yeast extract 5 g, Urea 1.5 g, KH₂PO₄ 4 g, K₂HPO₄ 8 g, MgSO₄·7H₂O 0.5 g, Biotin 0.1 mg, Thiamine HCl 1 mg, Calcium-pantothenic acid 22 mg, Nicotinamide 2 mg (based on 1 liter of distilled water)

### <Production medium (pH 7.2)>

Raw sugar 50 g, (NH₄)₂SO₄ 30 g, Yeast extract 1 g, KH₂PO₄ 1.1 g, MgSO₄·7H₂O 1.2 g, L-arginine 0.2 g, Biotin 1 mg, Thiamine hydrochloride 5 mg, Calcium-pantothenic acid 5 mg, Nicotinamide 15 mg, MnSO₄ 10 mg, FeSO₄ 10 mg, ZnSO₄ 0.5 mg, CuSO₄ 0.5 mg, CaCO₃ 30 g (based on 1 liter of distilled water)

**[Table 7]**

| Strain | | L-citrulline (g/l) | |
|---|---|---|---|
| | | 3BT mean | Improvement |
| Control | C.gl::argR*_argG* | 3.5 | - |
| | C.gl::argR*_argG*-Pcj7_aceF | 3.8 | 108% |
| | C.gl::argR*_argG*-Pcj7_aceF-Pcj7_aceE | 4.1 | 110% |

As shown in Table 7, it was confirmed that the citrulline-producing ability of the C. gl::argR*_argG*-Pcj7_aceF strain, in which pyruvate dehydrogenase complex dihydrolipoyllysine-residue acetyltransferase was enhanced with the Pcj7 promoter, increased by 108%, as compared to a parent citrulline-producing strain C. gl::argR*_argG*, and the citrulline-producing ability of the C. gl::argR*_argG*-Pcj7_aceF-Pcj7_aceE strain, in which pyruvate dehydrogenase complex subunit E1 was enhanced with the Pcj7 promoter in the C. gl::argR*_argG*-Pcj7_aceF strain, increased by 110%, as compared to a parent citrulline-producing strain C. gl::argR*_argG*-Pcj7_aceF. It was confirmed that the citrulline-producing ability of the C. gl::argR*_argG*-Pcj7_aceF-Pcj7_aceE strain increased by 117%, as compared to C. gl::argR*_argG*.

These results indicate that the enhancement of pyruvate dehydrogenase complex dihydrolipoyllysine-residue acetyltransferase in the citrulline-producing strains of the genus *Corynebacterium* was effective for citrulline production, and the additional enhancement of pyruvate dehydrogenase complex subunit E1 was effective for citrulline production.

### Example 4 : Preparation of ornithine-producing strain with enhanced activities of pyruvate dehydrogenase complex dihydrolipoyllysine-residue acetyltransferase and pyruvate dehydrogenase complex subunit E1 and Evaluation of ornithine-producing ability

### Example 4-1. Preparation of ornithine-producing strain

A strain for use in evaluating the ornithine-producing ability was prepared as follows.

### Example 4-1-1. Preparation of Shewanella oneidensis membrane

### protein-introduced and lysE-deleted strain

### Example 4-1-1-1. Construction of plasmid for introducing Shewanella

### oneidensis membrane protein

First, LysE/ArgO family amino acid transporter (WP_011072781.1) (SEQ ID NO: 48) which is a membrane protein derived from *Shewanella oneidensis* MR-1 was selected as a protein with high L-ornithine export activity, and a plasmid for introducing the *Shewanella oneidensis* membrane protein was constructed.

In order to amplify a nucleotide sequence of a gene encoding the protein, information on the gene encoding the membrane protein and the surrounding nucleic acid sequence (NC_004347.2) was obtained from the US NIH GenBank. DNA synthesis was performed based on the corresponding sequence information (Cosmo Genetech, Korea).

A vector for introducing the selected *Shewanella oneidensis* MR-1 membrane protein into the L-ornithine producing strain was constructed.

ANU34435.1, which is one of the transposases present inside the genome of wild-type *Corynebacterium glutamicum* ATCC 13869 (NZ_CP016335.1), was used as an insertion site. The genome of wild-type *Corynebacterium glutamicum* ATCC 13869 was used as a template to amplify the homologous recombinant A arm using a pair of primers of SEQ ID NOS: 49 and 50, and the homologous recombinant B arm using a pair of primers of SEQ ID NOS: 51 and 52.

In order to obtain a gene fragment encoding the membrane protein (SEQ ID NO: 48) derived from *Shewanella oneidensis* MR-1, PCR was performed using the synthesized DNA as a template and a pair of primers of SEQ ID NOS: 53 and 54.

To obtain the gapA promoter, PCR was performed using the genomic DNA of wild-type *Corynebacterium glutamicum* ATCC 13032 (NC_006958.1) as a template and a pair of primers of SEQ ID NOS: 55 and 56 by denaturation at 95°C for 2 min, followed by 25 cycles of denaturation at 95°C for 1 min, annealing at 55°C for 1 min, and polymerization at 72°C for 1 min, and then 72°C for 5 min. At this time, Solg^{™} Pfu-X DNA polymerase was used in PCR.

The amplified gapA promoter region, the gene fragment encoding the membrane protein derived from *Shewanella oneidensis* MR-1, the homologous recombinant arm gene fragment, and the vector pDCM2 (WO2021-187781 A1) digested with Sall and BamHI restriction enzymes were linked using the Gibson assembly method (DG Gibson et al., NATURE METHODS, VOL.6 NO.5, MAY 2009, NEBuilder HiFi DNA Assembly Master Mix), and then transformed into *E*. *coli* DH5α, and plated on an LB solid medium comprising kanamycin (25 mg/l).

PCR was performed using a pair of primers of SEQ ID NOS: 57 and 58 to select colonies which were transformed with the vector comprising the gene encoding the membrane protein derived from *Shewanella oneidensis* MR-1. The plasmid was extracted from the selected colonies using a plasmid prep kit (QIAGEN), and this plasmid was named pDCM2-PgapA-Son.

Information of the primers used is as listed in Table 8 below.

**[Table 8]**

| SEQ ID NO. | Sequence (5' -> 3') |
|---|---|
| 49 | CGGTACCCGGGGATCCTTTTAGATTCCC AGTGCAG |
| 50 | CGCGTGGGCTTTGGGCTGAG |
| 51 | TGCATTGGTGGAGCTAGCTG |
| 52 | ATGCCTGCAGGTCGACGCTATGCAGTGG AGATGTC |
| 53 | CTTTAGAGGAGACACAACATGCAAACGG CGTTTATTC |
| 54 | TAGCTCCACCAATGCATTAAATTGCGGCA AGGTAT |
| 55 | GCCCAAAGCCCACGCGAACGACCGAGC CTATTGGG |
| 56 | GTTGTGTCTCCTCTAAAGATTG |
| 57 | TATTACGCCAGCTGGCGAAA |
| 58 | GCTTTACACTTTATGCTTCC |

### Example 4-1-1-2. Construction of lysE deletion plasmid

Next, a plasmid for deleting lysE was constructed.

To construct a vector deleting the open reading frame (ORF) of wild-type *Corynebacterium glutamicum* LysE (ANU33473.1) (SEQ ID NO: 59), the genome of wild-type *Corynebacterium glutamicum* ATCC13869 was used as a template to amplify the homologous recombinant A arm using a pair of primers of SEQ ID NOS: 68 and 69, and the homologous recombinant B arm using a pair of primers of SEQ ID NOS: 70 and 71. The homologous recombinant arm gene fragments were cloned into the vector pDCM2 digested with Sall and BamHI restriction enzymes in the same manner as in Example 4-1-1-1, transformed into *E. coli* DH5α, and PCR was performed using a pair of primers of SEQ ID NOS: 57 and 58 in order to select transformed colonies. The plasmid was extracted from the selected colonies using the plasmid prep kit (QIAGEN), and this plasmid was named pDCM2-ΔlysE.

Information of the primers used is as follows.

**[Table 9]**

| SEQ ID NO. | Sequence (5' -> 3') |
|---|---|
| 68 | CGGTACCCGGGGATCCATTGCCTCACCA AAACCTTC |
| 69 | CGTGACCTATGGAAGTACTTAAG |
| 70 | GTACTTCCATAGGTCACGTTAGTTTTCGC GGGTTTTAG |
| 71 | ATGCCTGCAGGTCGACGTTTTGGTGGTT CCCGAAAG |

### Example 4-1-1-3. Construction of argF* plasmid

In order to prepare an L-ornithine-producing strain, a vector for substituting a stop codon for serine at position 55 from the N-terminus of an amino acid sequence of ArgF (ANU33618.1) (SEQ ID NO: 64) derived from wild-type *Corynebacterium glutamicum* was constructed. The genome of wild-type *Corynebacterium glutamicum* ATCC 13869 was used as a template to amplify the homologous recombinant A arm using a pair of primers of SEQ ID NOS: 60 and 61, and the homologous recombinant B arm using a pair of primers of SEQ ID NOS: 62 and 63. Thereafter, the plasmid was obtained in the same manner as in Example 4-1-1-1, and this plasmid was named pDCM2-argF(S55*).

### Example 4-1-1-4. Construction of C. gl::argF*_argR*_PgapA-Son and C. gl::argF*_argR*_ΔlysE_PgapA-Son strains

Wild-type *Corynebacterium glutamicum* ATCC 13869 was transformed using the pDCM2-argF(S55*) vector by electroporation (Appl. Microbiol. Biotechnol. (1999) 52:541-545), and through a secondary crossover process, a strain in which serine at position 55 from the N-terminus of the amino acid sequence of ArgF was replaced with a stop codon was obtained. PCR and nucleotide sequence analysis were performed using a pair of primers of SEQ ID NOS: 60 and 63 capable of amplifying the adjacent region comprising the position where the corresponding gene was inserted, and the corresponding genetic manipulation was confirmed and named C. gl::argF*.

C. gl::argF* was transformed with the pDCM2-argR(E47*) vector constructed in Example 3-1, and a strain in which glutamate at position 47 from the N-terminus of the amino acid sequence of ArgR was replaced with a stop codon was obtained in the same manner as above. PCR and nucleotide sequence analysis were performed using a pair of primers of SEQ ID NOS: 40 and 43 capable of amplifying the adjacent region comprising the position where the corresponding gene was inserted, and the corresponding genetic manipulation was confirmed. The strain thus obtained was named C. gl::argF*_argR*.

C. gl::argF*_argR* was transformed with the pDCM2-ΔlysE plasmid constructed in Example 4-1-1-2 by electroporation, and through a secondary crossover process, a strain in which the gene encoding LysE (lysE) was deleted was obtained. PCR and nucleotide sequence analysis were performed using a pair of primers of SEQ ID NOS: 68 and 71 capable of amplifying the adjacent region comprising the position where the corresponding gene was inserted, and the corresponding genetic manipulation was confirmed. The strain thus obtained was named C. gl::argF*_argR*_ΔlysE.

Information of the primers used is as follows.

**[Table 10]**

| SEQ ID NO. | Sequence (5' -> 3') |
|---|---|
| 60 | CGGTACCCGGGGATCCTGACCCCAGG CAAGCACGG |
| 61 | GAAGCGAGTACGAGTTTAAGTCTTATC |
| 62 | AAACTCGTACTCGCTTCTCC |
| 63 | ATGCCTGCAGGTCGACCGGCGCCGGC AACCTCGTC |

The pDCM2-PgapA-Son vector constructed in Example 4-1-1-1 was transformed into C. gl::argF*_argR* and the lysE-deleted strain, C. gl::argF*_argR*_ΔlysE, by electroporation, respectively, and then through a secondary crossover process, a strain in which PgapA-Son was inserted was obtained. PCR and nucleotide sequence analysis were performed using a pair of primers of SEQ ID NOS: 68 and 71 capable of amplifying the adjacent region comprising the position where the corresponding gene was inserted, and the corresponding genetic manipulation was confirmed.

The strains thus obtained were named C. gl::argF*_argR*_PgapA-Son and C. gl::argF*_argR*_ΔlysE_PgapA-Son.

### Example 4-1-2. Preparation of strain with weakened activity of carbamoyl phosphate synthase

### Example 4-1-2-1. Construction of vector for replacing carAB promoter

A vector for replacing the promoter of the carA gene encoding carbamoyl phosphate synthase small subunit (CarA) was constructed. Information on the carAB gene (SEQ ID NO: 67) encoding CarA (ANU33813.1, SEQ ID NO: 65) and the surrounding nucleic acid sequence (NZ_CP016335.1) was obtained from the US NIH GenBank, and they were used to construct a vector for replacing the promoter region of carA with the promoter of betP (ANU33153.1) (SEQ ID NO: 72).

The genome of wild-type *C*. *glutamicum* ATCC 13869 was used as a template to amplify the betP promoter using a pair of primers of SEQ ID NOS: 77 and 78, the homologous recombinant A arm using a pair of primers of SEQ ID NOS: 79 and 80, and the homologous recombinant B arm using a pair of primers of SEQ ID NOS: 81 and 82 by PCR (Sol^{™} Pfu-X DNA polymerase), and thus DNA fragments were obtained, respectively.

The obtained promoter and recombinant arm gene fragments were cloned into a vector pDCM2 (Korean Patent Publication No. 10-2020-0136813) digested with Sall and BamHI restriction enzymes using the Gibson assembly method (DG Gibson et al., NATURE METHODS, VOL.6 NO.5, MAY 2009, NEBuilder HiFi DNA Assembly Master Mix) to construct a vector. The constructed vector was transformed into *E. coli* DH5α and plated on an LB solid medium comprising kanamycin (25 mg/l). To select colonies transformed with the vector, PCR was performed using a pair of primers of SEQ ID NOS: 83 and 84, and plasmids were obtained from the selected colonies using a plasmid extraction method commonly known. The obtained plasmid was named pDCM2-PbetP-carA.

Information of the primers used is as follows.

**[Table 11]**

| SEQ ID NO. | Sequence (5' -> 3') |
|---|---|
| 77 | GGCACAAGATGGGGTGCGTGGCGAGA TCACCGTT |
| 78 | TATCGGTTTCGAATTTGGGTCAGATGTA GTCATA |
| 79 | CGGTACCCGGGGATCCCAAGAAGGCG |
| | CTTCTCG |
| 80 | GCACCCCATCTTGTGCCGGTGACTGTT GTCGCCT |
| 81 | CAAATTCGAAACCGATAGTGAGTAAAGA CACCAC |
| 82 | ATGCCTGCAGGTCGACGAGACCTCAAC GGAGAG |
| 83 | TATTACGCCAGCTGGCGAAA |
| 84 | GCTTTACACTTTATGCTTCC |

### Example 4-1-2-2. Construction of vector for carA start codon mutation

A vector for replacing a start codon gtg of the carA gene with ttg was constructed. The genome of wild-type C. *glutamicum* ATCC 13869 was used as a template to amplify the homologous recombinant A arm using a pair of primers of SEQ ID NOS: 79 and 85, and the homologous recombinant B arm using a pair of primers of SEQ ID NOS: 82 and 86. Then, the plasmid was obtained in the same manner as above, and named pDCM2-carA(g1t).

Information of the primers used is as follows.

**[Table 12]**

| SEQ ID NO. | Sequence (5' -> 3') |
|---|---|
| 85 | GGTGACTGTTGTCGCCTTTC GTGTGTCTGTC |
| 86 | GGCGACAACAGTCACCTTG AGTAAAGACACCAC |

### Example 4-1-2-3. Construction of vector for carB start codon mutation

A vector for replacing a start codon atg of the carB gene with ttg was constructed. Information on the carAB gene (SEQ ID NO: 67) encoding carbamoyl phosphate synthase large subunit (SEQ ID NO: 66) and the surrounding nucleic acid sequence (NZ_CP016335.1) was obtained from the US NIH GenBank, and the genome of wild-type *C*. *glutamicum* ATCC 13869 was used as a template to amplify the homologous recombinant A arm using a pair of primers of SEQ ID NOS: 73 and 74, and the homologous recombinant B arm using a pair of primers of SEQ ID NOS: 75 and 76. Then, the plasmid was obtained in the same manner as above, and named pDCM2-carB(a1t).

Information of the primers used is as follows.

**[Table 13]**

| SEQ ID NO. | Sequence (5' -> 3') |
|---|---|
| 73 | CGGTACCCGGGGATCCCCCACGTCGTTTCTCTGC AC |
| 74 | GTTATTTATGCGCCTTTCTTC |
| 75 | GAAAGGCGCATAAATAACTTGCCAAAGCGTTCAGA TAT |
| 76 | ATGCCTGCAGGTCGACGCCACCCATAGTGAAGGA TG |

### Example 4-1-2-4. Preparation of strain with weakened activity of carbamoyl phosphate synthase

The pDCM2-PbetP-carA and pDCM2-carA(g1t) vectors constructed in Examples 4-1-2-1 to 4-1-2-2 were transformed by electroporation into wild-type *C*. *glutamicum* ATCC 13869, and C. gl::argF* and C. gl::argF*_argR* which were constructed in 4-1-1-3, respectively, and then through a secondary crossover process, strains were obtained. PCR and nucleotide sequence analysis were performed using a pair of primers of SEQ ID NOS: 78 and 81, and the corresponding genetic manipulations were confirmed. The strains thus obtained were named C. gl::PbetP-carA, C. gl::carA(g1t), C. gl::argF*_PbetP-carA, C. gl::argF*_carA(g1t), C. gl::argF*_argR*_PbetP-carA, and C. gl::argF*_argR*_carA(g1t) in this order.

To prepare a strain in which the promoter and start codon of carB were replaced, the plasmid pDCM2-carB(a1t) vector obtained in Example 4-1-2-3 was transformed into wild-type *C*. *glutamicum* ATCC 13869, C. gl::argF*, C. gl::argF*_argR* by electroporation, respectively, and through a secondary crossover process, strains were obtained, respectively. PCR and nucleotide sequence analysis were performed using a pair of primers of SEQ ID NOS: 87 and 90 capable of amplifying the adjacent region comprising the position where the corresponding gene was inserted, and the corresponding genetic manipulation was confirmed. The strains thus obtained were named C. gl::carB(a1t), C. gl::argF*_carB(a1t), C. gl::argF*_argR*_carB(a1t) in this order.

### Example 4-2. Preparation of ornithine-producing strain with enhanced activities of pyruvate dehydrogenase complex dihydrolipoyllysine-residue acetyltransferase and pyruvate dehydrogenase complex subunit E1

In order to confirm whether or not the strain with enhanced activity has an effect of increasing the production ability in the *Corynebacterium glutamicum* strain with the L-ornithine-producing ability, strains were prepared by enhancing the activity of aceF in the *Corynebacterium glutamicum* L-ornithine-producing strains, C.gl::argF*_argR*_PgapA-Son and C.gl::argF*_argR*_ΔlysE_PgapA-Son, C.gl::argF*_argR*_carA(g1t) and C.gl::argF*_argR*_carB(a1t) prepared in Example 4-1.

In detail, the vector pDCM2-Pcj7_aceF constructed in Example 1 was transformed into *Corynebacterium glutamicum* C.gl::argF*_argR*_PgapA-Son, C.gl::argF*_argR*_ΔlysE_PgapA-Son, C.gl::argF*_argR*_carA(g1t), and C.gl::argF*_argR*_carB(a1t), respectively, by the homologous recombination on the chromosome (van der Rest et al., Appl Microbiol Biotechnol 52:541-545, 1999).

The recombinant strains were named C.gl::argF*_argR*_PgapA-Son-Pcj7_aceF, C.gl::argF*_argR*_ΔlysE_PgapA-Son-Pcj7_aceF, C.gl::argF*_argR*_carA(g1t)-Pcj7_aceF, and C.gl::argF*_argR*_carB(a1t)-Pcj7_aceF, respectively.

Further, the vector pDCM2-Pcj7_aceE constructed in Example 1-2, based on the strain with the enhanced aceF, was transformed into *Corynebacterium glutamicum* C.gl::argF*_argR*_PgapA-Son-Pcj7_aceF, C.gl::argF*_argR*_ΔlysE_PgapA-Son-Pcj7_aceF, C.gl::argF*_argR*_carA(g1t)-Pcj7_aceF, C.gl::argF*_argR*_carB(a1t)-Pcj7_aceF, respectively, by the homologous recombination on the chromosome (van der Rest et al., Appl Microbiol Biotechnol 52:541-545, 1999). The recombinant strains were named C.gl::argF*_argR*_PgapA-Son-Pcj7_aceF-Pcj7_aceE, C.gl::argF*_argR*_ΔlysE_PgapA-Son-Pcj7_aceF-Pcj7_aceE, C.gl::argF*_argR*_carA(g1t)-Pcj7_aceF-Pcj7_aceE, C.gl::argF*_argR*_carB(a1t)-Pcj7_aceF-Pcj7_aceE, respectively.

### Example 4-3. Evaluation of production ability of ornithine strain with enhanced activities of pyruvate dehydrogenase complex dihydrolipoyllysine-residue acetyltransferase and pyruvate dehydrogenase complex subunit E1

To compare the ornithine-producing ability between ornithine-producing strains prepared in Example 4-2, a flask test was performed. First, each strain was inoculated into a 250 ml corner-baffled flask comprising 25 ml of a seed medium, and cultured at 30°C for 20 hours with shaking at 200 rpm. 1 ml of the seed culture was inoculated into a 250 ml corner-baffled flask comprising 24 ml of a production medium, and cultured at 33°C for 42 hours with shaking at 200 rpm. After the culturing was completed, the production amount of L-ornithine was measured by HPLC, and the results are shown in Table 14 below.

### <Seed medium (pH 7.0)>

Glucose 20 g, Peptone 10 g, Yeast extract 5 g, Urea 1.5 g, KH₂PO₄ 4 g, K₂HPO₄ 8 g, MgSO₄·7H₂O 0.5 g, Biotin 0.1 mg, Thiamine HCl 1 mg, Calcium-pantothenic acid 22 mg, Nicotinamide 2 mg (based on 1 liter of distilled water)

### <Production medium (pH 7.0)>

Raw sugar 50 g, (NH₄)₂SO₄ 25 g, Yeast extract 1 g, KH₂PO₄ 0.55 g, MgSO₄·7H₂O 0.6 g, L-arginine 0.2 g, Biotin 0.9 mg, Thiamine hydrochloride 4.5 mg, Calcium-pantothenic acid 4.5 mg, Nicotinamide 30 mg, MnSO₄ 9 mg, FeSO₄ 9 mg, ZnSO₄ 0.45 mg, CuSO₄ 0.45 mg, CaCO₃ 30 g (based on 1 liter of distilled water)

**[Table 14]**

| Strain | L-ornithine (g/l) | |
|---|---|---|
| | 3BT mean | Improvemen t |
| C. glutamicum WT | 0 | - |
| C.gl::argF*_argR* | 17.2 | - |
| C.gl::argF*_argR*_PgapA-Son | 20.3 | - |
| C.gl::argF*_argR*_ΔlysE_PgapA-Son | 19.1 | - |
| C.gl::argF*_argR*_PbetP-carA | 20.9 | - |
| C.gl::argF*_argR*_carA(g1t) | 20.1 | - |
| C.gl::argF*_argR*_carB(a1t) | 20.5 | - |
| C.gl::argF*_argR*_PgapA-Son-Pcj7_aceF | 22.7 | 112% |
| C.gl::argF*_argR*_ΔlysE_PgapA-Son-Pcj7_aceF | 21.6 | 113% |
| C.gl::argF*_argR*_PbetP-carA-Pcj7 Pcj7 aceF | 24.0 | 115% |
| C.gl::argF*_argR*_parA(g1t)-Pcj7_Pcj7 aceF | 22.7 | 113% |
| C.gl::argF*_argR*_carB(a1t)-Pcj7_aceF | 24.6 | 112% |
| C.gl::argF*_argR*_carB(a1t)-Pcj7_aceF-Pcj7 aceE | 25.4 | 112% |
| C.gl::argF*_argR*_PgapA-Son-Pcj7_**aceF**-Pcj7 aceE | 23.9 | 111% |
| C.gl::argF*_argR*_ΔlysE_PgapA-Son-Pcj7_**aceF**-Pcj7 aceE | 27.3 | 114% |
| C.gl::argF*_argR*_PbetP-carA-Pcj7 aceE-Pcj7 aceE | 25.4 | 112% |
| C.gl::argF*_argR*_carA(g1t)-Pcj7_**aceF**- Pcj7_aceE | 27.5 | 112% |

As shown in Table 14, it was confirmed that the L-ornithine-producing ability of the L-ornithine-producing strain, in which pyruvate dehydrogenase complex dihydrolipoyllysine-residue acetyltransferase (aceF) was enhanced, increased by 112% to 115%, as compared to the parent L-ornithine-producing strain. It was also confirmed that the ornithine-producing ability of the L-ornithine-producing strain, in which pyruvate dehydrogenase complex subunit E1 (aceE) was further enhanced based on the strain with enhanced pyruvate dehydrogenase complex dihydrolipoyllysine-residue acetyltransferase (aceF), increased by 111% to 114%, as compared to the parent strain in which pyruvate dehydrogenase complex dihydrolipoyllysine-residue acetyltransferase (aceF) was enhanced, and increased by 125% to 134%, as compared to the parent strain.

These results indicate that the enhancement of pyruvate dehydrogenase complex dihydrolipoyllysine-residue acetyltransferase in the L-arginine-producing strains of the genus *Corynebacterium* was effective for L-ornithine production, and the additional enhancement of pyruvate dehydrogenase complex subunit E1 activity was also effective for L-ornithine production.

### Example 5 : Preparation of O-acetyl homoserine- and homoserine-producing strain with enhanced activities of pyruvate dehydrogenase complex dihydrolipoyllysine-residue acetyltransferase and pyruvate dehydrogenase complex subunit E1 and Evaluation of production ability

### Example 5-1. Preparation of O-acetyl homoserine- and homoserine-producing strain

### Example 5-1-1. metB deletion

The *metB* gene encoding cystathionine gamma-synthase of the O-acetyl homoserine degradation pathway was obtained through PCR using chromosomal DNA of *Corynebacterium glutamicum* ATCC13032 as a template. Information of the nucleotide sequence (NCBI accession number Ncgl2360, SEQ ID NO: 91) of the *metB* gene was obtained from the US NIH GenBank, and based on this, primers (SEQ ID NOS: 87 and 88) comprising the N-terminal part and the linker part of the *metB* gene and primers (SEQ ID NOS: 89 and 90) comprising the C-terminal part and the linker part were synthesized. The primer sequences are listed in Table 15 below.

**[Table 15]**

| SEQ ID NO. | Sequence name | Sequence |
|---|---|---|
| 87 | metB_N_del_F | GGCTTCGGAGTTGGAGCG |
| 88 | metB_N_del R | GCCAAATAGTTTcccgggGGTAGATCAACTCCT GTAATCA |
| 89 | metB_C_del F | CAGGAGTTGATCTACCcccgggAAACTATTTG GCGGCAAG |
| 90 | metB_C_del R | TCGCGTCTGGGTGCGCATC |

PCR was performed using chromosomal DNA of ATCC13032 as a template and primers of SEQ ID NOS: 87 and 88 and SEQ ID NOS: 89 and 90. PfuUltra^{™} high-fidelity DNA polymerase (Stratagene) was used as a polymerase, and the PCR conditions consisted of 30 cycles of denaturation at 96°C for 30 sec, annealing at 53°C for 30 sec, and polymerization at 72°C for 1 min. As a result, 558 bp of the amplified gene comprising the N-terminal part and the linker part of the metB gene and 527 bp of the amplified gene comprising the C-terminal part and the linker part of the metB gene were obtained, respectively.

PCR was performed using the two amplified genes obtained above as templates, and the PCR conditions consisted of 10 cycles of denaturation at 96°C for 60 sec, annealing at 50°C for 60 sec, and polymerization at 72°C for 1 min, and then SEQ ID NOS: 92 and 95 were added and the polymerization reaction was further repeated 20 times. As a result, a 1064-bp inactivation cassette comprising the N-terminal-linker-C-terminal of the metB gene was obtained.

The pDCM2 vector was treated with Smal, and the PCR product (1064 bp) obtained above was fusion-cloned with the pDCM2 vector treated with Smal restriction enzyme using In-Fusion^{®} HD Cloning Kit (Clontech). The cloned vector was transformed into *E. coli* DH5α, and the transformed *E. coli* was plated on an LB solid medium comprising 25 mg/l kanamycin. From the LB medium, colonies transformed with the plasmid were selected, and then the plasmid was obtained using a plasmid extraction method (US Patent No. US 5981235 A), and finally, a recombinant vector pDCM2-ΔmetB was constructed, in which the *metB* gene deletion cassette was cloned.

The constructed pDCM2-ΔmetB vector was transformed into ATCC13032ΔNCgl2335::PCJ7-yjeH(eco,F351L) (KCCM12634P; WO2021-125896 A1) strain by an electric pulse method, and through a secondary crossover process, ATCC13032ΔNCgl2335::PCJ7-yjeH(eco,F351L)ΔmetB was obtained, in which the *metB* gene on the chromosome was inactivated. The inactivation of the *metB* gene was finally confirmed by comparing with ATCC13032, in which the *metB* gene was not inactivated, after PCR using primers of SEQ ID NOS: 87 and 90.

### Example 5-1-2. metY deletion

The *metY* gene encoding O-acetylhomoserine (thiol)-lyase of the O-acetylhomoserine degradation pathway was obtained through PCR using chromosomal DNA of *Corynebacterium glutamicum* ATCC13032 as a template. Information on the nucleotide sequence of the *metY* gene (NCBI accession number Ncgl0625, SEQ ID NO: 92) was obtained from the US NIH GenBank, and based on this, primers comprising the N-terminal part and the linker part of the *metY* gene (SEQ ID NOS: 93 and 94) and primers comprising the C-terminal part and the linker part (SEQ ID NOS: 95 and 96) were synthesized. The primer sequences are as shown in Table 16 below.

**[Table 16]**

| SEQ ID NO. | Sequence name | Sequence |
|---|---|---|
| 93 | metY_N del F | CCAAAGACAAGGAGACCA |
| 94 | metY_N_del R | AGTCTATTTAAAGcccgggTTGGAGGTCCTT AAGAGTTTT |
| 95 | metY_C_del F | TAAGGACCTCCAAcccgggCTTTAAATAGAC TCACCCCAG |
| 96 | metY_C_del R | ATGCCGAGTGCGTCGAGG |

PCR was performed using chromosomal DNA of ATCC13032 as a template and primers of SEQ ID NOS: 93 and 94 and SEQ ID NOS: 95 and 96. PfuUltra^{™} high-fidelity DNA polymerase (Stratagene) was used as a polymerase, and the PCR conditions consisted of 30 cycles of denaturation at 96°C for 30 sec, annealing at 53°C for 30 sec, and polymerization at 72°C for 1 min. As a result, 548 bp of the amplified gene comprising the N-terminal part and the linker part of the *metY* gene and 550 bp of the amplified gene comprising the C-terminal part and the linker part of the *metY* gene were obtained, respectively. PCR was performed using the two amplified genes obtained above as templates, and the PCR conditions consisted of 10 cycles of denaturation at 96°C for 60 sec, annealing at 50°C for 60 sec, and polymerization at 72°C for 1 min, and then SEQ ID NOS: 97 and 100 were added and the polymerization reaction was further repeated 20 times. As a result, a 1077-bp inactivation cassette comprising the N-terminal-linker-C-terminal of the *metY* gene was obtained.

The pDCM2 vector was treated with Smal, and the PCR product (1077 bp) obtained above was fusion-cloned with the pDCM2 vector treated with Smal restriction enzyme using In-Fusion^{®} HD Cloning Kit (Clontech). The cloned vector was transformed into *E. coli* DH5α, and the transformed *E. coli* was plated on an LB solid medium comprising 25 mg/l kanamycin. From the LB medium, colonies transformed with the plasmid were selected, and then the plasmid was obtained using a plasmid extraction method (US Patent No. US 5981235 A), and finally, a recombinant vector pDCM2-ΔmetY was constructed, in which the *metY* gene deletion cassette was cloned.

The constructed pDCM2-ΔmetY vector was transformed into ATCC13032 ΔNCgl2335::PCJ7-yjeH(eco,F351L) ΔmetB by an electric pulse method, and through a secondary crossover process, ATCC13032 ΔNCgl2335::PCJ7-yjeH(eco,F351L) ΔmetB ΔmetY was obtained, in which the metY gene on the chromosome was further inactivated. The inactivation of the *metY* gene was finally confirmed by comparing with ATCC13032, in which the *metY* gene was not inactivated, after PCR using primers of SEQ ID NOS: 93 and 96.

### Example 5-1-3. lysC(L377K) introduction

A mutation (L377K) (US 10662450 B2) for enhancing the expression of the lysC gene and for releasing feedback inhibition by L-lysine and L-threonine was introduced into the lysC gene (SEQ ID NO: 97) encoding aspartokinase derived from *Corynebacterium glutamicum* ATCC13032. To prepare a vector comprising the mutant lysC gene, a pair of primers (SEQ ID NOS: 98 and 99) for amplifying the 5' upstream region, centered around the mutation position, and a pair of primers (SEQ ID NOS: 100 and 101) for amplifying the 3' downstream region were designed. The primer sequences are shown in Table 17 below.

**[Table 17]**

| SEQ ID NO. | Sequence name | Sequence |
|---|---|---|
| 98 | lysC_L377K_5 F | CAGAAGCTGGAAAAGCTCA |
| 99 | lysC_L377K_5 R | ATCTCAGAGGTGGAAATCttTTCGATGTTCACG TTGACAT |
| 100 | lysC_L377K_3 F | TGTCAACGTGAACATCGAAaaGATTTCCACCT CTGAGATT |
| 101 | lysC_L377K. 3 R | TCCTTGTCGGAAGGGTTCA |

PCR was performed using the chromosome of ATCC13032 as a template, and primers of SEQ ID NO: 98 and SEQ ID NO: 99, and SEQ ID NO: 100 and SEQ ID NO: 101. The PCR conditions consisted of denaturation at 95°C for 5 min, followed by 30 cycles of denaturation at 95°C for 30 sec, annealing at 55°C for 30 sec, and polymerization at 72°C for 30 sec, and then polymerization at 72°C for 7 min. As a result, a 512-bp DNA fragment of the 5' upstream region and a 522-bp DNA fragment of the 3' downstream region, centered around the mutation site of the lysC gene, were obtained.

PCR was performed using the two amplified DNA fragments as templates and primers of SEQ ID NO: 98 and SEQ ID NO: 101. The PCR conditions consisted of denaturation at 95°C for 5 min, followed by 30 cycles of denaturation at 95°C for 30 sec, annealing at 55°C for 30 sec, and polymerization at 72°C for 60 sec, and then polymerization at 72°C for 7 min. As a result, a 1011-bp DNA fragment comprising the mutant lysC (L377K) gene encoding the aspartokinase mutant, in which the 377^{th} leucine was replaced with lysine, was amplified.

The pDCM2 vector was treated with Smal, and the PCR product (1011 bp) obtained above was fusion-cloned with the pDCM2 vector treated with Smal restriction enzyme using the In-Fusion^{®} HD Cloning Kit (Clontech). The cloned vector was transformed into *E. coli* DH5α, and the transformed *E. coli* was plated on an LB solid medium comprising 25 mg/l kanamycin. From the LB medium, colonies transformed with the plasmid were selected, and the plasmid was obtained using a plasmid extraction method (US Patent No. US 5981235 A), and finally, a pDCM2-lysC(L377K) recombinant vector was constructed, into which the cassette with substitution of the lysC (L377K) gene was cloned.

The constructed pDCM2-lysC(L377K) vector was transformed into the strain ATCC13032 ΔNCgl2335::PCJ7-yjeH(eco,F351L) ΔmetB ΔmetY by an electric pulse method, and through a secondary crossover process, *Corynebacterium glutamicum* ATCC13032 ΔNCgl2335::PCJ7-yjeH(eco,F351L) ΔmetB ΔmetY lysC(L377K) was obtained, in which the nucleotide mutation was introduced into the lysC gene on the chromosome. The introduction of the nucleotide mutation was finally confirmed by comparing with the sequence of the wild-type lysC gene through sequencing, after PCR using primers of SEQ ID NOS: 98 and 101.

### Example 5-1-4. NCgl0616 deletion

A vector for deleting an endogenous gene NCgl0616 (SEQ ID NO: 38) in *Corynebacterium glutamicum* ATCC13032 was constructed.

In detail, a pair of primers (SEQ ID NOS: 102 and 103) for amplifying the 5' upstream region and a pair of primers (SEQ ID NOS: 104 and 105) for amplifying the 3' downstream region, centered around the position of the NCgl0616 gene of SEQ ID NO: 38, were designed.

**[Table 18]**

| SEQ ID NO. | Sequence name | Sequence |
|---|---|---|
| 102 | 0616 _del up F | TCGAGCTCGGTACCCAGCATAATTCCATACT CCCAC |
| 103 | 0616 _del up R | GGTAGCGCTTGGGCTGCAGTACTCTCGACG GCGTGCTCAATT |
| 104 | 0616 _del down F | AATTGAGCACGCCGTCGAGAGTACTGCAGC CCAAGCGCTACC |
| 105 | 0616 _del down R | CTCTAGAGGATCCCCCTACGACGCTACTGC GCAG |

PCR was performed using the chromosome of ATCC13032 wild-type (WT) as a template, and primers of SEQ ID NO: 102 and SEQ ID NO: 103, SEQ ID NO: 104 and SEQ ID NO: 105. The PCR conditions consisted of denaturation at 95°C for 5 min, followed by 30 cycles of denaturation at 95°C for 30 sec, annealing at 55°C for 30 sec, and polymerization at 72°C for 30 sec, and then polymerization at 72°C for 7 min. As a result, a 701-bp DNA fragment of the 5' upstream region and a 699-bp DNA fragment of the 3' downstream region, centered around the deletion site of the NCgl0616 gene, were obtained.

PCR was performed using the two amplified DNA fragments as templates and primers of SEQ ID NO: 102 and SEQ ID NO: 105. The PCR conditions consisted of denaturation at 95°C for 5 min, followed by 30 cycles of denaturation at 95°C for 30 sec, annealing at 55°C for 30 sec, and polymerization at 72°C for 90 sec, and then polymerization at 72°C for 7 min. As a result, a 1410-bp DNA fragment comprising the site capable of deleting the NCgl0616 gene was amplified.

The pDCM2 vector was treated with Smal, and the PCR product (2912 bp DNA fragment) obtained above was fusion-cloned using the pDCM2 vector treated with Smal restriction enzyme and the In-Fusion^{®} HD Cloning Kit (Clontech). The cloned vector was transformed into *E. coli* DH5α, and the transformed *E. coli* was plated on an LB solid medium comprising 25 mg/l kanamycin. From the LB medium, colonies transformed with the plasmid were selected, and the plasmid was obtained using a plasmid extraction method (US Patent No. US 5981235 A), and finally, a pDCM2-ΔNCgl0616 recombinant vector was constructed, into which the NCgl0616 deletion cassette was cloned.

**[Table 19]**

| SEQ ID NO. | Sequence name | Sequence |
|---|---|---|
| 106 | 0616 del F | GGCTTCCAACTGTAATGG |
| 107 | 0616_del R | TAGAACACCCAGCTAACA |

The constructed pDCM2-ΔNCgl0616 vector was transformed into the strain with enhanced O-acetyl homoserine-producing ability by an electric pulse method, and through a secondary crossover process, ATCC13032 ΔNCgl2335::PCJ7-yjeH(eco,F351L) ΔmetB ΔmetY lysC(L377K) ΔNCgl0616 was obtained, in which the NCgl0616 gene on the chromosome was deleted.

The inactivation of the NCgl0616 gene was finally confirmed by comparing with ATCC13032, in which the NCgl0616 gene was not inactivated, after PCR using primers of SEQ ID NOS: 106 and 107.

### Example 5-2. Preparation of O-acetyl homoserine- and homoserine-producing strain with enhanced activities of pyruvate dehydrogenase complex dihydrolipoyllysine-residue acetyltransferase and pyruvate dehydrogenase complex subunit E1

In order to confirm whether or not the strain with enhanced activity has an effect of increasing the production ability in the *Corynebacterium glutamicum* strain with the O-acetyl homoserine- and homoserine-producing ability, strains were prepared by enhancing the activity of pyruvate dehydrogenase complex dihydrolipoyllysine-residue acetyltransferase (NCBI accession number NCgl2126, hereinafter, referred to as NCgl2126 (SEQ ID NO: 32) gene number *Corynebacterium glutamicum* ATCC13032-derived NCgl2126 (SEQ ID NO: 33)) in the *Corynebacterium glutamicum* O-acetyl homoserine- and homoserine-producing strain ATCC13032 ΔNCgl2335::PCJ7-yjeH(eco,F351L) ΔmetB ΔmetY lysC(L377K)ΔNCgl0616 prepared in Example 5-1, and its effectiveness was determined.

In detail, to construct a vector for enhancing pyruvate dehydrogenase complex dihydrolipoyllysine-residue acetyltransferase, the Pcj7 promoter (US 7662943 B2) (SEQ ID NO: 3) which is known as a strong promoter was used to replace the wild-type promoter of the aceF gene with Pcj7, thereby constructing a plasmid for enhancing the NCgl2126 activity. Upstream and downstream regions of the NCgl2126 gene were obtained by performing PCR. In detail, to prepare a strain into which the Pcj7 promoter was introduced, PCR was performed using chromosomal DNA of *Corynebacterium glutamicum* ATCC13032 as a template to amplify gene fragments of the upstream region of the NCgl2126 gene using primers of SEQ ID NO: 4 and SEQ ID NO: 5, and the downstream region of the NCgl2126 gene using primers of SEQ ID NO: 6 and SEQ ID NO: 34, respectively. In addition, the Pcj7 promoter fragment was obtained using pDCM2-Pcj7 as a template and SEQ ID NO: 8 and SEQ ID NO: 9.

PfuUltra^{™} high-fidelity DNA polymerase (Stratagene) was used as a polymerase for the PCR reaction, and the PCR conditions consisted of denaturation at 95°C for 30 sec; annealing at 53°C for 30 sec; and polymerization at 72°C for 1 min. Denaturation, annealing, and polymerization of these conditions were repeated for 28 cycles. As a result, a 318-bp DNA fragment of the Pcj7 promoter region, a 526-bp upstream DNA fragment and a 529-bp downstream DNA fragment of *Corynebacterium glutamicum* ATCC13032 NCgl2126 were obtained, respectively. PCR was performed using the amplified promoter and DNA fragments as templates and primers of SEQ ID NO: 4 and SEQ ID NO: 34. The PCR conditions consisted of denaturation at 95°C for 5 min, followed by 28 cycles of denaturation at 95°C for 30 sec; annealing at 55°C for 30 sec; and polymerization at 72°C for 2 min, and then polymerization at 72°C for 5 min. The two fragments obtained above were subjected to DNA purification, and fusion cloning was performed according to the provided manual using In-Fusion^{®} HD cloning kit (Clontech) and Smal restriction enzyme-treated pDCM2 vector (WO2021-187781 A1) to obtain a plasmid. The resulting vector was named pDCM2-Pcj7_NCgl2126, and the sequences and detailed information of the primers used in this Example are listed in Table 20.

**[Table 20]**

| SEQ ID NO. | Sequence name | Sequence |
|---|---|---|
| 34 | NCgl2126-3'-R | GGTCGACTCTAGAGGATCCCACCTTGTCGGTG GAGACCTCAA |

The pDCM2-Pcj7_NCgl2126 vector constructed above was transformed into *Corynebacterium glutamicum* strain ATCC13032 ΔNCgl2335::PCJ7-yjeH(eco,F351L) ΔmetB ΔmetY lysC(L377K)ΔNCgl0616 by homologous recombination on the chromosome (van der Rest et al., Appl Microbiol Biotechnol 52:541-545, 1999). The recombinant strain ATCC13032ΔNCgl2335::PCJ7-yjeH(eco,F351L) ΔmetB ΔmetY lysC(L377K)ΔNCgl0616 -Pcj7_NCgl2126 was named CM04-8000.

Further, the vector pDCM2-Pcj7_aceE constructed in Example 1-2 was transformed into CM04-8000 strain (van der Rest et al., Appl Microbiol Biotechnol 52:541-545, 1999). The recombinant strain (ATCC13032ΔNCgl2335::PCJ7-yjeH(eco,F351L) ΔmetB ΔmetY lysC(L377K)ΔNCgl0616 -Pcj7_NCgl2126-Pcj7_aceE) was named CM04-8002.

### Example 5-3. Evaluation of production ability of O-acetyl homoserine and homoserine strain with enhanced activities of pyruvate dehydrogenase complex dihydrolipoyllysine-residue acetyltransferase and pyruvate dehydrogenase complex subunit E1

A flask test was performed to compare production abilities between O-acetyl homoserine and homoserine-producing strains, *Corynebacterium glutamicum* ATCC13032 ΔNCgl2335::PCJ7-yjeH(eco,F351L) ΔmetB ΔmetY lysC(L377K)ΔNCgl0616, CM04-8000, and CM04-8002.

One inoculation loop of each strain was inoculated into a 250 ml corner-baffled flask comprising 25 ml of the following medium, and cultured at 33°C for 20 hours with shaking at 200 rpm. The O-acetyl homoserine concentrations were analyzed using HPLC, and the analyzed concentrations are shown in Table 21.

### O-acetyl homoserine production medium (pH 7.2)

Glucose 30 g, KH₂PO₄ 2 g, urea 3 g, (NH₄)₂SO₄ 40 g, peptone 2.5 g, CSL (Sigma) 5 g (10 ml), MgSO₄·7H₂O 0.5 g, CaCO₃ 20 g (based on 1 liter of distilled water)

**[Table 21]**

| Strains | O-AH (g/L) |
|---|---|
| ATCC13032 | 0.27 |
| ATCC13032ΔNCgl2335::PCJ7-yjeH(eco,F351L) ΔmetB ΔmetY lysC(L377K)ΔNCgl0616 | 1.73 |
| CM04-8000 (ATCC13032 ΔNCgl2335::PCJ7-yjeH(eco,F351L) ΔmetB ΔmetY lysC(L377K)ΔNCgl0616-Pcj7_NCgl2126) | 1.90 |
| CM04-8002 (ATCC13032 ΔNCgl2335::PCJ7-yjeH(eco,F351L) ΔmetB ΔmetY lysC(L377K)ΔNCgl0616-Pcj7_NCgl2126-Pcj7_aceE) | 2.14 |

As shown in Table 21, it was confirmed that the O-acetyl homoserine concentration increased by about 110% to 124% in the O-acetyl homoserine-producing strains with enhanced activities of pyruvate dehydrogenase complex dihydrolipoyllysine-residue acetyltransferase and pyruvate dehydrogenase complex subunit E1, as compared to the O-acetyl homoserine-producing strain.

### Example 5-4. Construction of plasmid for introducing O-acetyl homoserine transferase (MetX)

In order to amplify the gene encoding O-acetyl homoserine transferase (MetX), the nucleotide sequence information of the metX gene (NCBI accession number NCgl0624, SEQ ID NO: 35) was obtained from the US NIH GenBank, and based on this, primers (SEQ ID NOS: 36 and 37) were designed to amplify from the promoter region (300 bp upstream of the start codon) to the terminator region (100 bp downstream of the stop codon), and BamHI restriction enzyme sites were inserted at both ends of the primer sequence. The PCR conditions consisted of denaturation at 95°C for 5 min, followed by 30 cycles of denaturation at 95°C for 30 sec, annealing at 55°C for 30 sec, and polymerization at 72°C for 90 sec, and then polymerization at 72°C for 7 min. As a result, a 1546-bp DNA fragment of the coding region of the metX gene was obtained. The pECCG117 (KR 10-0057684) vector and the metX DNA fragment were treated with the restriction enzyme BamHI, and ligated using a DNA ligase, and then cloned to obtain a plasmid, which was named pECCG117-metX WT. The sequences of the primers are shown in Table 22 below.

**[Table 22]**

| SEQ ID NO. | Sequence name | Sequence |
|---|---|---|
| SEQ ID NO: 36 | metX F | GGATCCCCTCGTTGTTCACCCAGCAACC |
| SEQ ID NO: 37 | metX R | GGATCCCAAAGTCACAACTACTTATGTTA G |

The constructed pECCG117-metX WT vector was introduced into ATCC13032 ΔNCgl2335::PCJ7-yjeH(eco,F351L) ΔmetB ΔmetY lysC(L377K)ΔNCgl0616 and CM04-8000 strains prepared in Example 5-2 by an electric pulse method, and then plated on a selection medium comprising 25 mg/l kanamycin to obtain each transformant.

To compare the O-acetyl homoserine-producing ability between the strains prepared above, they were cultured by the following method, and O-acetyl homoserine in the culture was analyzed.

One inoculation loop of each strain was inoculated into a 250 ml corner-baffled flask comprising 25 ml of the following medium, and cultured at 33°C for 20 hours with shaking at 200 rpm. The O-acetyl homoserine concentrations were analyzed using HPLC, and the analyzed concentrations are shown in Table 23.

### O-acetyl homoserine production medium (pH 7.2)

Glucose 30 g, KH₂PO₄ 2 g, urea 3 g, (NH₄)₂SO₄ 40 g, peptone 2.5 g, CSL (Sigma) 5 g (10 ml), MgSO₄·7H₂O 0.5 g, methionine 400 mg, CaCO₃ 20 g (based on 1 liter of distilled water)

**[Table 23]**

| Strains | O-AH (g/L) |
|---|---|
| ATCC13032ΔNCgl2335::PCJ7-yjeH(eco,F351L) ΔmetB ΔmetY lysC(L377K)ΔNCgl0616 | 1.73 |
| CM04-8000 (ATCC13032 ΔNCgl2335::PCJ7-yjeH(eco,F351L) ΔmetB ΔmetY lysC(L377K)ΔNCgl0616 -Pcj7 NCgl2126) | 1.90 |
| CM04-8002 (ATCC13032 ΔNCgl2335::PCJ7-yjeH(eco,F351L) ΔmetB ΔmetY lysC(L377K)ΔNCgl0616 -Pcj7_NCg12126-Pcj7_aceE) | 2.14 |
| ATCC13032 ΔNCgl2335::PCJ7-yjeH(eco,F351L) ΔmetB ΔmetY lysC(L377K)ΔNCgl0616) **/pECCG117-metX WT** | 2.83 |
| CM04-8000 (ATCC13032 ΔNCgl2335::PCJ7-yjeH(eco,F351L) ΔmetB ΔmetY lysC(L377K)ΔNCgl0616 -Pcj7_NCgl2126) **/pECCG117-metX WT** | 3.25 |
| CM04-8002 (ATCC13032 ΔNCgl2335::PCJ7-yjeH(eco,F351L) ΔmetB ΔmetY lysC(L377K)ΔNCgl0616 -Pcj7 NCgl2126-Pcj7_aceE) **/pECCG117-metX WT** | 3.68 |

As a result, as shown in Table 23, it was confirmed that when the ATCC13032 ΔNCgl2335::PCJ7-yjeH(eco,F351L) ΔmetB ΔmetY lysC(L377K) ΔNCgl0616/pECCG117-metX WT strain was cultured, 2.83 g/L of O-acetyl-L-homoserine was accumulated, and when CM04-8002 was cultured, O-acetyl-L-homoserine was 3.68 g/L, indicating that the production amount increased by 130%. When the activity of pyruvate dehydrogenase complex dihydrolipoyllysine-residue acetyltransferase was enhanced in the O-acetyl-L homoserine-producing strains, the O-acetyl-L-homoserine-producing ability was increased, and when the activity of pyruvate dehydrogenase complex subunit E1 was further enhanced, the O-acetyl-L-homoserine-producing ability was also increased.

These results indicate that the enhancement of pyruvate dehydrogenase complex dihydrolipoyllysine-residue acetyltransferase activity was effective for O-acetyl-L-homoserine and homoserine production in the O-acetyl-L-homoserine- and homoserine-producing strains of the genus *Corynebacterium,* and the additional enhancement of pyruvate dehydrogenase complex subunit E1 was also effective for O-acetyl-L-homoserine and homoserine production.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

## Claims

1. An L-amino acid-producing microorganism with increased activity of pyruvate dehydrogenase complex dihydrolipoyllysine-residue acetyltransferase (AceF), as compared to its endogenous activity.

2. The microorganism of claim 1, wherein the L-amino acid is any one or more selected from glutamine, glutamate, ornithine, citrulline, arginine, proline, O-acetylhomoserine, homoserine, and methionine.

3. The microorganism of claim 1, wherein the microorganism is a microorganism of the genus *Corynebacterium.*

4. The microorganism of claim 3, wherein the microorganism of the genus *Corynebacterium* is *Corynebacterium glutamicum.*

5. The microorganism of claim 1, wherein the pyruvate dehydrogenase complex dihydrolipoyllysine-residue acetyltransferase is derived from *Corynebacterium glutamicum.*

6. The microorganism of claim 1, wherein the pyruvate dehydrogenase complex dihydrolipoyllysine-residue acetyltransferase comprises an amino acid sequence of SEQ ID NO: 1 or SEQ ID NO: 32.

7. The microorganism of claim 6, wherein the pyruvate dehydrogenase complex dihydrolipoyllysine-residue acetyltransferase is encoded by a polynucleotide of SEQ ID NO: 2 or SEQ ID NO: 33.

8. The microorganism of any one of claims 1 to 7, wherein the activity of pyruvate dehydrogenase complex subunit E1 is increased, as compared to its endogenous activity.

9. The microorganism of claim 8, wherein the pyruvate dehydrogenase complex subunit E1 comprises an amino acid sequence of SEQ ID NO: 10.

10. The microorganism of claim 9, wherein the pyruvate dehydrogenase complex subunit E1 is encoded by a polynucleotide of SEQ ID NO: 11 or SEQ ID NO: 39.

11. A method of producing an L-amino acid, the method comprising the step of culturing, in a medium, a microorganism with increased activity of pyruvate dehydrogenase complex dihydrolipoyllysine-residue acetyltransferase (AceF), as compared to its endogenous activity.

12. The method of claim 11, wherein the L-amino acid is any one or more selected from glutamine, glutamate, ornithine, citrulline, arginine, proline, O-acetylhomoserine, homoserine, and methionine.

13. The method of claim 9, wherein the microorganism has increased activity of pyruvate dehydrogenase complex subunit E1, as compared to its endogenous activity.

14. The method of claim 9, further comprising the step of recovering the L-amino acid from the cultured microorganism, a culture of the microorganism, a fermentation product of the microorganism, or the culture medium.

15. A composition for producing an L-amino acid, the composition comprising a microorganism with increased activity of pyruvate dehydrogenase complex dihydrolipoyllysine-residue acetyltransferase, as compared to its endogenous activity, a culture of the microorganism, a fermentation product of the microorganism, or a combination of two or more thereof.
